# EUROPEAN PATENT SPECIFICATION

(11) **EP 1 567 199 B1**
(45) Date of publication and mention of the grant of the patent: **23.07.2014**
(21) Application number: 03790377.0
(22) Date of filing: 05.12.2003
(51) Int. Cl.: A61K 38/00, G01N 33/50

(54) **NEUROPROTECTIVE ACTIVITY OF ACTIVATED PROTEIN C IS INDEPENDENT OF ITS ANTICOAGULANT ACTIVITY**
NEUROPROTEKTIVE WIRKUNG VON AKTIVIERTEM PROTEIN C IST UNABHÄNGIG VON SEINER GERINNUNGSHEMMENDEN WIRKUNG
ACTIVITE NEUROPROTECTRICE DE LA PROTEINE C ACTIVEE INDEPENDANTE DE SON ACTIVITE ANTICOAGULANTE

(30) Priority: 05.12.2002 US 430936 P; 24.01.2003 US 442066 P; 25.04.2003 US 465235 P
(43) Date of publication of application: 31.08.2005
(73) Proprietor: ZZ BIOTECH L.L.C., Houston, TX 77002 (US); The University of Rochester, Rochester, NY 14627 (US); THE SCRIPPS RESEARCH INSTITUTE, La Jolla, CA 92037 (US)
(72) Inventor: ZLOKOVIC, Berislav, V., Rochester, NY 14610 (US); GRIFFIN, John, H., Del Mar, CA 92014 (US)
(74) Representative: Predazzi, Valentina
(86) International application number: PCT/US2003/038764
(87) International publication number: WO 2004/056309

(56) References cited:
- WO-A-01/56532
- WO-A-01/72328
- WO-A-98/42358
- US-A1- 2002 028 199
- US-A1- 2004 033 517
- SHIBATA M ET AL: "ANTI-INFLAMMATORY, ANTITHROMBOTIC, AND NEUROPROTECTIVE EFFECTS OF ACTIVATED PROTEIN C IN A MURINE MODEL OF FOCAL ISCHEMIC STROKE" CIRCULATION, LIPPINCOTT WILLIAMS & WILKINS, US, vol. 103, 1 April 2001 (2001-04-01), pages 1799-1805, XP002941848 ISSN: 0009-7322
- HIROSE K ET AL: "Activated protein C reduces the ischemia/reperfusion-induced spinal cord injury in rats by inhibiting neutrophil activation." ANNALS OF SURGERY AUG 2000, vol. 232, no. 2, August 2000 (2000-08), pages 272-280, XP002527577 ISSN: 0003-4932
- TAOKA Y ET AL: "The long-term effects of pre-treatment with activated protein C in a rat model of compression-induced spinal cord injury" SPINAL CORD, vol. 38, no. 12, December 2000 (2000-12), pages 754-761, XP009116755 ISSN: 1362-4393
- BERNARD G R ET AL: "EFFICACY AND SAFETY OF RECOMBINANT HUMAN ACTIVATED PROTEIN C FOR SEVERE SEPSIS" NEW ENGLAND JOURNAL OF MEDICINE, THE, MASSACHUSETTS MEDICAL SOCIETY, WALTHAM, MA, US, vol. 344, no. 10, 8 March 2001 (2001-03-08), pages 699-709, XP009019299 ISSN: 0028-4793
- CHENG T ET AL: "Activated Protein C Blocks p53-Mediated Apoptosis in Ischemic Human Brain Endothelium and its Neuroprotective" NATURE MEDICINE, NATURE PUBLISHING GROUP, NEW YORK, NY, US, vol. 9, no. 3, 1 March 2003 (2003-03-01), pages 338-342, XP002998642 ISSN: 1078-8956
- GUO HUANG ET AL: "Activated protein C prevents neuronal apoptosis via protease activated receptors 1 and 3." NEURON, vol. 41, no. 4, 19 February 2004 (2004-02-19), pages 563-572, XP002527579 ISSN: 0896-6273
- GRIFFIN J H ET AL: "The promise of protein C" BLOOD CELLS, MOLECULES AND DISEASES, LAJOLLA, US, vol. 36, no. 2, 1 March 2006 (2006-03-01), pages 211-216, XP024917746 ISSN: 1079-9796 [retrieved on 2006-03-01]
- GRIFFIN JOHN H ET AL: "Activated protein C: potential therapy for severe sepsis, thrombosis, and stroke" 20020701; 20020700, vol. 39, no. 3, 1 July 2002 (2002-07-01), pages 197-205, XP008075814
- JOYCE D E ET AL: "Gene expression profile of antithrombotic protein C defines new mechanisms modulating inflammation and apoptosis" JOURNAL OF BIOLOGICAL CHEMISTRY, AMERICAN SOCIETY OF BIOLOCHEMICAL BIOLOGISTS, BIRMINGHAM, US, vol. 276, no. 14, 6 April 2001 (2001-04-06), pages 11199-11203, XP002973994 ISSN: 0021-9258
- RIEWALD M ET AL: "Activation of endothelial cell protease activated receptor 1 by the protein C pathway" SCIENCE, AMERICAN ASSOCIATION FOR THE ADVANCEMENT OF SCIENCE, US, WASHINGTON, DC, vol. 296, no. 5574, 7 June 2002 (2002-06-07), pages 1880-1882, XP002409975 ISSN: 0036-8075
- GALE ANDREW J; ET AL: "Molecular characterization of an extended binding site for coagulation factor Va in the positive exosite of activated protein C", JOURNAL OF BIOLOGICAL CHEMISTRY, vol. 277, no. 32, 9 August 2002 (2002-08-09), pages 28836-28840, XP002532145,
- GLASSCOCK L N; ET AL: 'Basic residues in the 37-loop of activated protein C modulate inhibition by protein C inhibitor but not by alpha1-antitrypsin' BIOCHIMICA ET BIOPHYSICA ACTA vol. 1649, no. 1, 26 June 2003, pages 106 - 117
- TAOKA Y ET AL: "ACTIVATED PROTEIN C REDUCES THE SEVERITY OF COMPRESSION-INDUCED SPINAL CORD INJURY IN RATS BY INHIBITING ACTIVATION OF LEUKOCYTES", JOURNAL OF NEUROSCIENCE, NEW YORK, NY, US, vol. 18, no. 4, 15 February 1998 (1998-02-15), pages 1393-1398, XP000995795, ISSN: 0270-6474

## Description

### FEDERALLY-SPONSORED RESEARCH OR DEVELOPMENT

The U.S. Government has certain rights in this invention as provided by NIH grants HL63290 and HL52246 from the Department of Health and Human Services.

### BACKGROUND OF THE INVENTION

This invention relates to the use of activated protein C (APC), prodrug, and/or a variant thereof as defined in the claims as an inhibitor of apoptosis or cell death and/or a cell survival factor, especially for cells or tissues of the nervous system which are stressed or injured. This biological function of APC can be separated from its anticoagulant function (i.e., inhibition of clotting). The invention can be used to treat a ischemia and stroke by inhibiting the p53-signaling pro-apoptotic pathway in stressed or injured brain cells. Here, this was shown in human brain endothelium *in vitro* and in animals *in vivo* (ischemic stroke and NMDA models). APC may act via the endothelial protein C receptor (EPCR) and the protease activated receptor-1 (PAR-1) on stressed brain endothelial cells, or the PAR-1 and the protease activated receptor-3 (PAR-3) on stressed neurons, to activate anti-apoptotic pathways and/or pro-survival pathways in these stressed and/or injured brain cells.

Protein C was originally identified for its anticoagulant and profibrinolytic activities. Upon activation of the zymogen form, activated protein C (APC) is a serine protease which deactivates Factors Vₐ and VIIIₐ. Human protein C is primarily made in the liver as a single polypeptide of 461 amino acids. This precursor molecule is then post-translationally modified by (i) cleavage of a 42 amino acid signal sequence, (ii) proteolytic removal from the one-chain zymogen of the lysine residue at position 155 and the arginine residue at position 156 to produce the two-chain form (i.e., light chain of 155 amino acid residues attached by disulfide linkage to the serine protease-containing heavy chain of 262 amino acid residues), (iii) carboxylation of the glutamic acid residues clustered in the first 42 amino acids of the light chain resulting in nine gamma-carboxyglutamic acid (Gla) residues, and (iv) glycosylation at four sites (one in the light chain and three in the heavy chain). The heavy chain contains the serine protease triad of Asp257, His211 and Ser360.

Similar to most other zymogens of extracellular proteases and the coagulation factors, protein C has a core structure of the chymotrypsin family, having insertions and an N-terminus extension that enable regulation of the zymogen and the enzyme. Of interest are two domains with amino acid sequences similar to epidermal growth factor (EGF). At least a portion of the nucleotide and amino acid sequences for protein C from human, monkey, mouse, rat, hamster, rabbit, dog, cat, goat, pig, horse, and cow are known, as well as mutations and polymorphisms of human protein C (see GenBank accession P04070). Other variants of human protein C are known which affect different biological activities.

Recently drotrecogin alfa (activated) (recombinant human activated protein C) has been approved for use as a treatment of sepsis. Although its efficacy may be due in part to APC's antithrombotic activity, Grinnell and his colleagues propose that its effects in sepsis could be attributed to APC's anti-inflammatory and cell survival activities.

The ability of activated protein C to suppress proinflammatory pathways and cellular survival mechanisms at the endothelial-mononuclear cell interface suggests a complex adaptive response at the vessel wall to protect the organism from vascular insult and to prolong endothelial, cellular, and organ survival. Joyce & Grinnell, *Crit. Care Med.* **30**:S288-S293 (2002). Based on transcriptional profiling, staurosporine-induced apoptosis in human umbilical vein endothelial cells (HUVEC), and tumor necrosis factor-α-mediated injury of HUVEC, Joyce et al. (J. Biol. Chem. 276:11199-11203, 2001) suggest that APC's inhibition of NF-κB signaling causes down regulation of adhesion molecules, while the induction of anti-apoptotic genes (e.g., Bcl2-related protein A1 or Bcl2A1, inhibitor of apoptosis 1 or clAP1, endothelial nitric oxide synthase or eNOS) has been interpreted as a possible mechanism linked to APC's anti-apoptotic effects in a staurosporine model. But the expression of these genes was not studied there. The direct role of these genes in the staurosporine model has yet to be shown and other reports do not suggest their major protective role (Ackerman et al., J. Biol. Chem. 274:11245-11252, 1999). Also no data on brain endothelial cells were obtained nor were the studies on cellular stress generalized to any other inducers of apoptosis. The role of these anti-apoptotic genes were not shown in any of the model; the only direct effect of APC which was determined was inhibition of NF-κB signaling. It is of note that NF-κB is a transcription factor which may have a dual function in the nervous system and endothelium, and could be anti-apoptotic or pro-apoptotic (Ryan et al. Nature 404:892-897, 2000; Yu et al. J. Neurosci. 19:8856-8865, 1999; Yabe et al. J. Biol. Chem. 276:43313-43319, 2001). Therefore the cell survival effects or cytoprotection via down regulation of NF-κB in the nervous system and vascular system does not explain direct cellular protective effects of APC. These observations were also limited to the staurosporine model or a TNF-α mechanism in HUVEC.

Taylor & Esmon (U.S. Patent 5,009,889) disclose that APC inhibits the inflammatory stimuli which disrupt cell permeability and normal coagulation processes in a patient suffering from dysfunction of the vascular endothelium. They were concerned with treating a systemic disorder of endothelial cells in sepsis (presumably by reducing the inflammatory response), instead of preventing apoptosis or promoting cell survival. They also did not suggest treatment of neurodegenerative diseases or the prevention of neuronal cell death and injury. ,

Griffin et al. (U.S. Patent 5,084,274) and Grinnell et al. (U.S. Patent 6,037,322) disclose that APC may be used to treat thrombotic occlusion or thromboembolism, which could involve stroke. Again, no data in a stroke model was provided nor were the effects of APC on neurological or neuropathological outcome determined. These patents were concerned with APC's anticoagulant activity in treating thrombosis. But they did not suggest direct neuroprotective cellular effects of APC, nor that anticoagulant activity was not required for this effect. It is possible that this anticoagulant activity may limit APC's use in treating stroke due to bleeding complications. In contrast, we show this activity is not critical for neuroprotection.

Griffin et al. (WO 01/56532) discloses that APC may be used for the treatment of neuropathological disorders and brain inflammatory diseases. It shows that APC provides neuroprotection in a murine model of focal cerebral ischemia.

Riewald et al. (Science 296:1880-1882, 2002) reported that APC uses the endothelial cell protein C receptor (EPCR) as a coreceptor for activation of protease activated receptor-1 (PAR-1) on endothelial cells. They also found Bcl2A1 and clAP1 are upregulated. However, their results on endothelium were limited to HUVEC and activation of mitogen-activated protein kinase (MAPK) phosphorylation. In addition, there are significant differences in cellular responses and their regulation of gene expression between HUVEC and brain endothelial cells, or any other type of brain cells (Berger et al., Molec. Med. 5:795-805, 1999; Petzelbauer et al. J. Immunol. 151:5062-5072, 1993; Abbot et al., Arthritis & Rheumatism 35:401-406, 1992; Mason et al., Am. J. Physiol. 273:C1233-C1240, 1997).

The prior art did not show the value of brain endothelium as a therapeutic target in stroke or ischemia. APC's vascular targets EPCR and PAR-1, and neuronal targets PAR-1 and PAR-3, which are linked intracellularly to down regulation of p53 under ischemic conditions and/or during overstimulation of neuronal N-methyl-D-aspartate (NMDA) receptors both *in vitro* and *in vivo,* were not suggested to promote cell survival in the brain. Moreover, the anti-apoptotic pathway of APC during hypoxia and brain endothelial cell injury which we describe is distinct from the induction of anti-apoptotic genes by APC in HUVEC previously described by Joyce et al. and Riewald et al. Similar, activation of the anti-apoptotic pathway by APC in injured or stressed neurons has not been previously described.

The prior art's use of APC in stroke was concerned with treating thrombosis, instead of achieving neuroprotection by directly protecting brain endothelium and neurons from ischemic cell death or excitotoxic cell death. Our *in vitro* and *in vivo* studies demonstrate that APC's neuroprotective effects do not depend on its anticoagulant activity. Moreover, our use of a stroke model confirms that APC's anticoagulant activity is not required for neuroprotection, and our use of a model of brain excitotoxic lesions *in vivo* indicates that systemic effects of APC are not required for its direct neuronal protective effects.

We have previously shown in patent application Serial Nos. 09/777,484 and PCT/US01/03758 that APC can be used as a neuroprotective agent by virtue of its action to prevent transmigration of leukocytes across the blood-brain barrier (BBB) into brain parenchyma during an ischemic insult to the brain. Thus, reduction of the inflammatory component of an ischemic injury was associated with neuroprotection. Here, in a stroke model in which a species homologous APC is used (i.e., murine recombinant APC administered to a mouse), neuroprotection with low dose APC (0.02 mg/kg/min for 1 min) is achieved in the absence of a significant decrease of neutrophils in brain tissue or at least does not depend on reduction of the number of leukocytes in ischemic brain tissue. Moreover, our results with APC in an *in vivo* NMDA model of brain excitotoxic lesions confirm that APC exerts direct neuronal protective effects and that its neuroprotective effects do not depend on its anticoagulant and anti-inflammatory effects associated with the reduction of the number of leukocytes in injured brain tissue.

It was also suggested that direct cell survival effects on ischemia-injured cells cannot be excluded. We now demonstrate that under normal culturing conditions, the anti-apoptotic effect during ischemia does not depend on the increased expression of the anti-apoptotic genes which were studied by Riewald et al. and Joyce et al. Here, we show that APC inhibits apoptosis of ischemic human brain vascular endothelium through a combination of down regulation of the p53-mediated mechanism and the Bax/Bcl-2 ratio, and inhibition of caspase-3 signaling. We have also confirmed in cultured neurons that APC blocks NMDA-induced neuronal apoptosis by reducing p53-dependent and caspase-3-dependent pro-apoptotic signaling. Use of activated protein C at low doses with reduced or no anticoagulant effects to inhibit apoptosis and/or as a cell survival factor can be separated from other functions like inhibiting thrombosis and leukocyte infiltration. For example, a low dose of APC may be administered to provide neuroprotection, and the neuroprotective effect does not depend on APC's anticoagulant properties. This shows that APC's anticoagulant activity is not required for APC-mediated neuroprotection.

It is an objective of the invention to use activated protein C (APC), prodrugs, and variants comprised of at least the mutation KKK191-193AAA as neuroprotective agents to inhibit p53-mediated apoptosis in brain cells as a result of a ischemia and stroke and/or to act as cell survival factors by inhibiting p53-mediated programmed cell death in brain cells or, more particularly, brain vascular endothelium.

An adverse effect of treatment with drotrecogin alfa (activated) is bleeding (see Lyseng-Williamson & Perry. Drugs 62:617-630, 2002). Thus, another objective of the invention is to provide variant products (e.g., mutant APC comprised of at least the mutation KKK191-193AAA) or processes (e.g., low dose) to reduce its anticoagulant and anti-thrombotic activities and, ultimately, the incidence or severity of bleeding.

A long-felt need for new therapeutic and prophylactic pharmaceutical compositions is addressed thereby. Also provided are therapeutic and prophylactic methods for inhibition of apoptosis or cell death and promotion of cell survival. Variants of protein C (i.e., a prodrug), variants of activated protein C, formulation strategies, and treatment protocols may be selected for their effect on the p53 signaling pathway or ability to act via EPCR and PAR-1 on endothelium, and PAR-1 and PAR-3 on neurons. Processes for using and making the aforementioned products are described. Further objectives and advantages of the invention are described below.

### SUMMARY OF THE INVENTION

The present invention is directed toa pharmaceutical composition as defined in the claims and to the use of a functional variant of an activated protein C (APC) or a human protein C as defined in the claims.. An effective amount of activated protein C (APC), at least one prodrug (e.g., protein C and variants thereof), or at least one variant thereof (e.g., APC protease domain mutants with reduced anticoagulant activity) comprised of at least the mutation KKK191-193AAA may be used to provide at least neuroprotection, to inhibit apoptosis or cell death, and/or to promote cell survival in stressed or injured brain cells and, more particularly, in stressed or injured brain endothelium and neurons.

Therefore, the invention provides a treatment for therapy or prophylaxis of ischemia and stroke, and the products used therein. Pharmaceutical compositions may be manufactured and assessed in accordance therewith. Further aspects of the invention will be apparent to persons skilled in the art from the following detailed description and claims, and generalizations thereto.

### BRIEF DESCRIPTIONS OF THE DRAWINGS

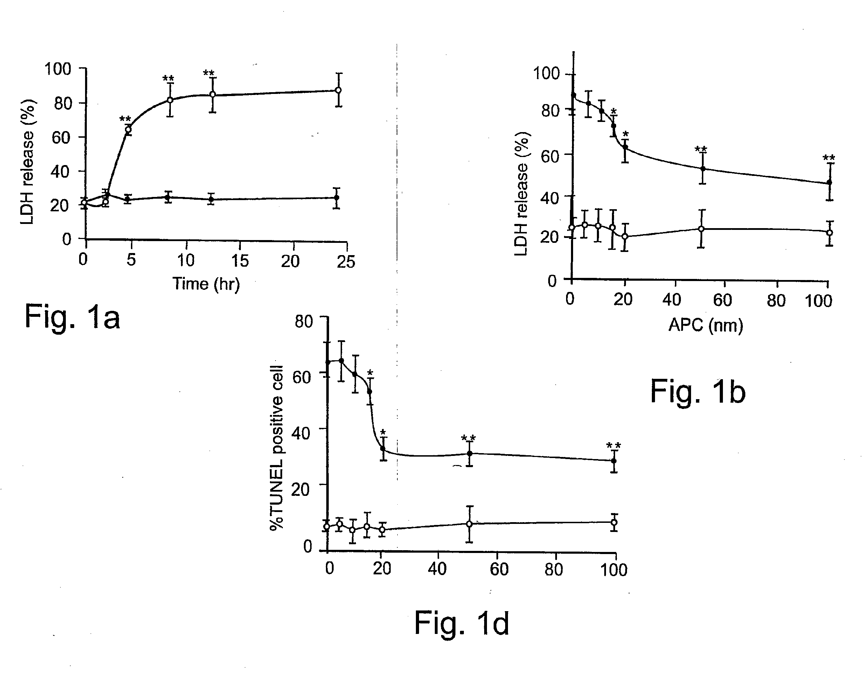

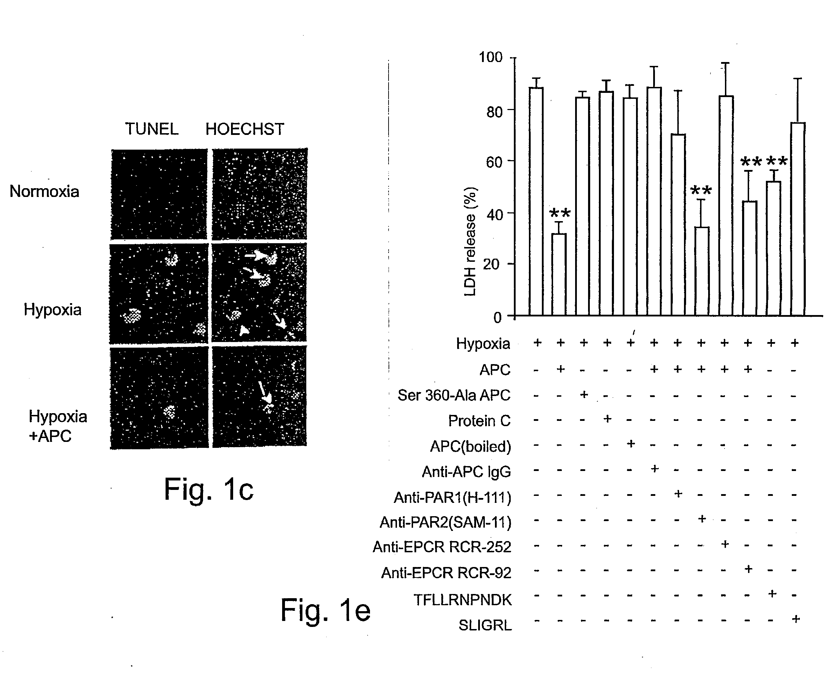

Figures 1a-1e show the anti-apoptotic effects of APC in hypoxic human brain endothelial cells (BEC). Fig. 1a, LDH release from BEC was time dependent under hypoxic and normoxic conditions. Fig. 1b, Cytoprotective effect of APC was dose dependent in hypoxic BEC and had no effect on normoxic BEC. Fig. 1c, TUNEL (left) and Hoechst staining (right) was performed simultaneously in normoxic BEC (upper panels) and hypoxic BEC in the absence (middle panels) and presence of 100 nM APC (lower panels). Fig.1d, Percentage of TUNEL-positive BEC under hypoxic and normoxic conditions versus APC is shown. Fig. 1e, Effects of reagents on hypoxic BEC injury (left to right): buffer alone, APC (100 nM), Ser360Ala-APC (100 nM)¹⁷, protein C zymogen (100 nM), boiled APC (100 nM). APC (100 nM) with antibodies: anti-APC IgG (C3, 11 µg/ml)²⁶, anti-PAR-1 (H-111, 20 µg/ml), anti-PAR-2 (SAM-11, 20 µg/ml), anti-EPCR (RCR-252, 15 µg/ml)¹⁸ and (RCR-92, 15 µg/ml)¹⁸ are shown. For Figs. 1*a*-1*b* and 1*d*-1*e*, data are mean ± SD (3-5 independent measurements in triplicate for each time point); * *P* < 0.05 and ** *P* < 0.01. For Fig. 1a, hypoxia values were compared to normoxia values; for Figs. 1b and 1d-1e, hypoxia values in the presence of APC or other studied molecular reagents were compared with values in the absence of APC.

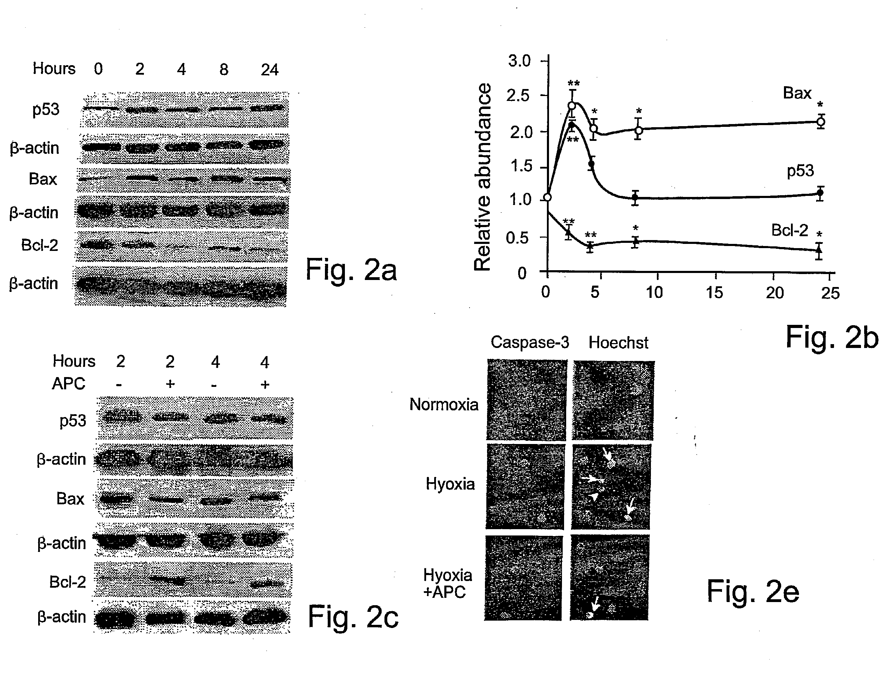

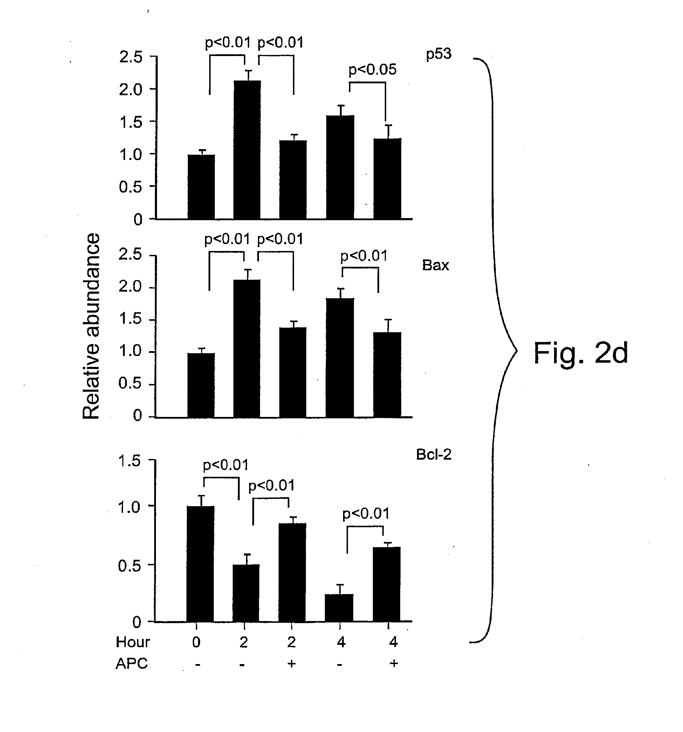

Figures 2a-2e show that APC blocks p53-dependent apoptosis in hypoxic human BEC. Fig. 2a, Time course of p53, Bax, or Bcl-2 protein expression in hypoxic BEC was studied by Western blot analysis. Fig. 2b, The intensity of p53, Bax, or Bcl-2 signal during hypoxia was determined by scanning densitometry and normalized to β-actin. Protein abundance was expressed relative to zero time whose value was arbitrarily taken as 1. Figs. 2c-2d, APC (100 nM) effects on p53, Bax, or Bcl-2 expression in hypoxic BEC were studied by Western blotting and densiometry (as in Figs. 2a-2b) at 2 hr and 4 hr. APC effects remain persistent within 24 hr. Fig. 2e, Immunostaining for the active form of caspase-3 under normoxic (upper panel, left) and hypoxic conditions in the absence (middle panel, left) or the presence of 100 nM APC (lower panel, left) was performed simultaneously with the Hoechst staining (right panels). For Figs. 2b and 2d, data are mean ± SD, from 3 to 5 independent measurements performed for each time point; * *P* < 0.05 and ** *P* < 0.01.

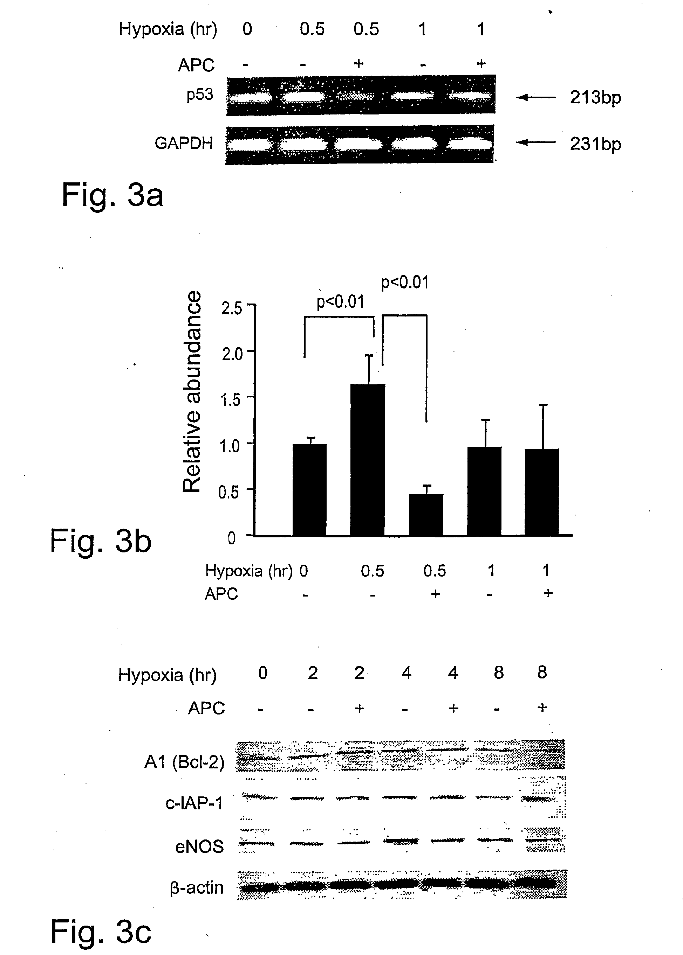

Figures 3a-3b show that APC inhibits p53 transcription in hypoxic BEC. Fig. 3a, Agarose gel electrophoresis of the PCR products corresponding to p53 and GAPDH (internal control) mRNA transcripts in normoxic and hypoxic BEC in the absence and presence of 100 nM APC is shown. Fig. 3b, Relative abundance of p53 mRNA normalized by GAPDH in hypoxic BEC in the absence or the presence of APC is shown. Data are mean ± SD, from 4 independent measurements performed for each time point with P < 0.01 where indicated. Fig. 3c, APC (100 nM) does not affect the expression of Bcl2-related protein A1, clAP1, or eNOS in hypoxic BEC studied by Western blot analysis (as in Figure 2).

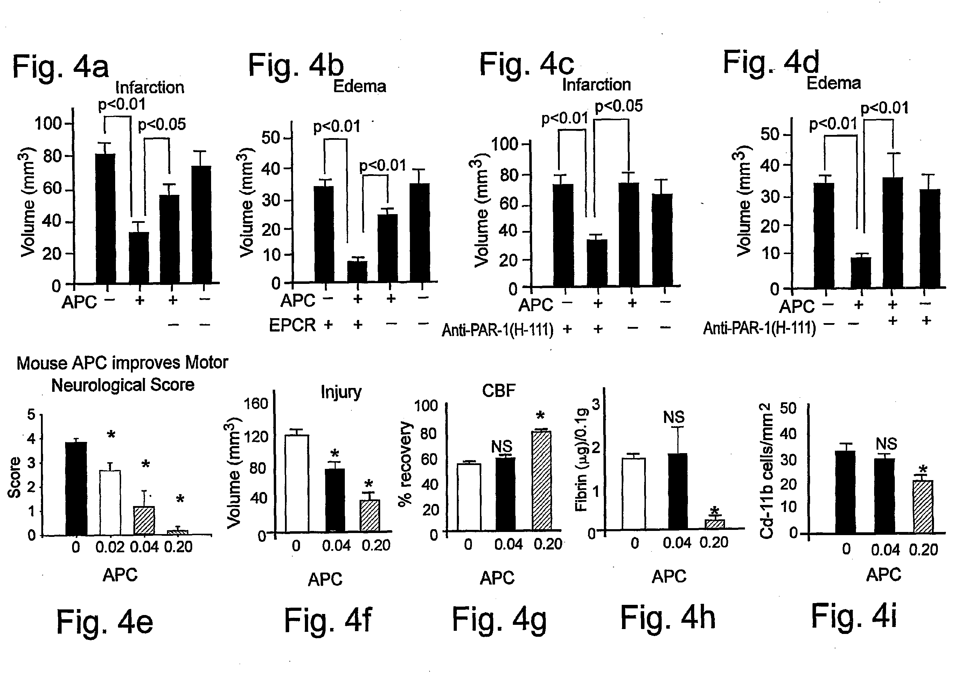

Figures 4a-4i show that *in vivo* neuroprotective effects of murine (mouse) recombinant APC during focal ischemic stroke in mice require EPCR and PAR-1. Figs. 4a-4b, Infarction and edema volumes in mice with a severe deficiency of EPCR (EPCR-) treated with APC (APC+) or vehicle (APC-), and in genetically-matched normal littermate controls (EPCR+) treated with either APC (APC+) or vehicle (APC-) are shown. Mean ± SE, n = 6. APC (0.2 mg/kg) was administered 10 min after the MCAO. Figs. 4c-4d, Infarction and edema volume in C57BL/6 mice treated with the anti-PAR-1 antibodies [anti-PAR-1 (H111)+] in the presence of APC (APC+) or absence of APC (APC-), and in control mice [anti-PAR-1 (H111)-] treated with either APC (APC+) or vehicle (APC-) are shown. Anti-PAR-1 antibodies (40 µg per mice i.v.) were administered 10 min prior to the MCAO; APC (0.2 mg/kg) was given 10 min after the MCAO. Mean ± SE, n = 6. Figs. 4e-4i, Motor neurological score, total brain injury volume (infarction + edema), post-ischemic cerebral blood flow (CBF), fibrin deposition, and the number of CD11b-positive leukocytes in mice subjected to focal ischemia by the MCAO treated with either vehicle (0) or APC (0.02, 0.04, or 0.20 mg/kg/min infused over 1 min) are shown. Values are mean ± SE, n = 6.

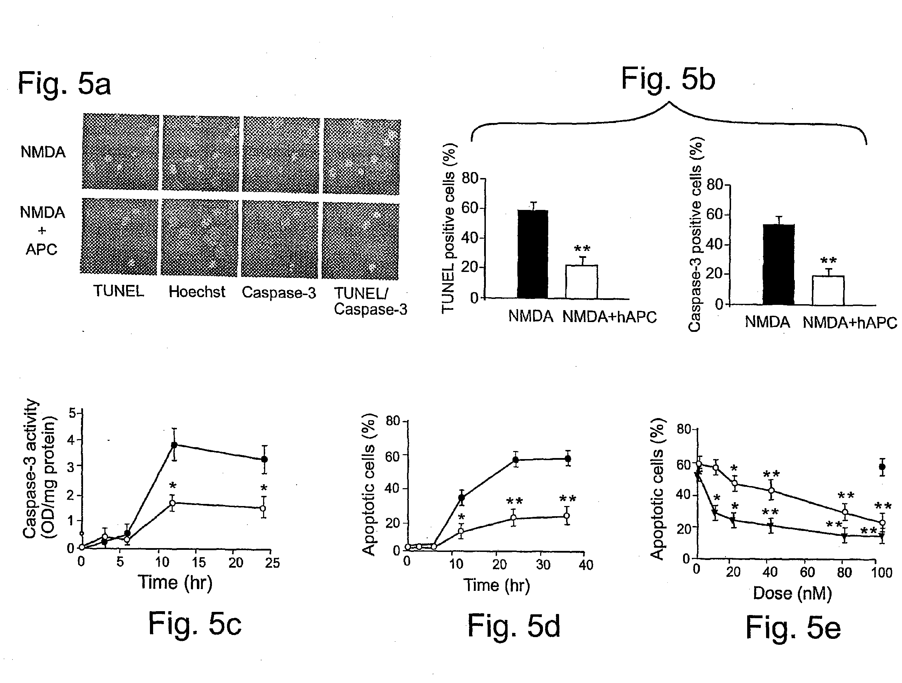

Figures 5a-5e show the protective effects of APC on NMDA-induced apoptosis in cultured mouse cortical neurons. Fig. 5a, Immunostaining for active caspase-3 in cortical neurons 24 hr after exposure to NMDA in the absence or presence of human APC (100 nM) was performed simultaneously with TUNEL and Hoechst staining. Fig. 5b, The number of TUNEL-positive cells (left) and caspase-3 positive cells (right) in experiments illustrated in Fig. 5a expressed as the percentage of total number of Hoechst positive nuclei. Fig. 5c, Time-dependent changes in caspase-3 activity in cultured cortical neurons were determined after exposure to NMDA in the absence (filled circle) or presence (open circle) of human APC (100 nM). Fig. 5d, Neuroprotective effect of human APC (100 nM) on NMDA-induced neuronal apoptosis shown as a function of time; the number of apoptotic cells was quantitated using TUNEL and Hoechst staining. Filled circle, NMDA only; open circle, NMDA and human APC. Fig. 5e, Human APC (hAPC) and recombinant mouse APC (mAPC) had dose-dependent effects on cortical neurons 24 hr after exposure to NMDA. Filled circle, NMDA only; open circle, NMDA and human APC; triangle, NMDA and mouse APC. Data are mean ± SEM (3-5 independent measurements in triplicate). ** *P* < 0.01, * *P* < 0.05 compared with values in the absence of APC.

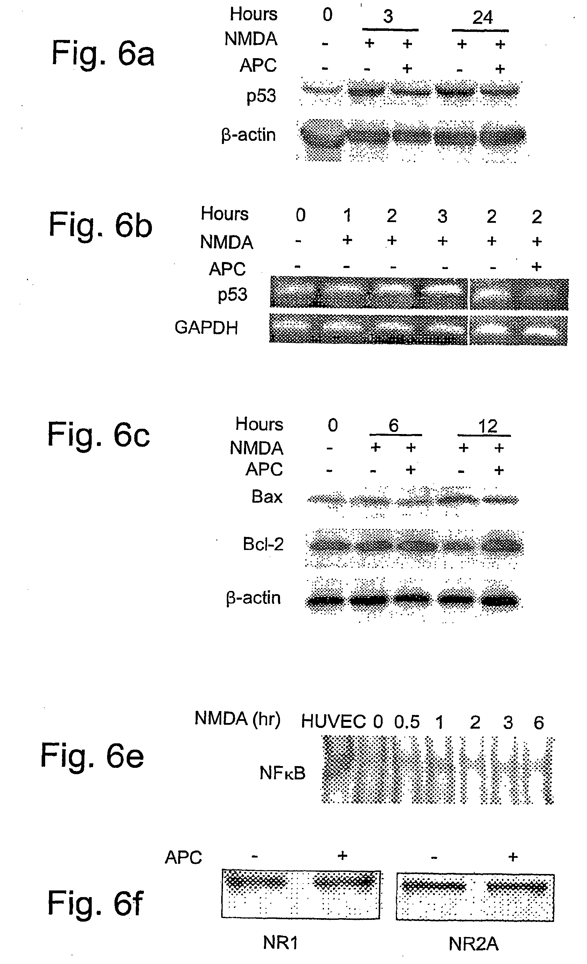

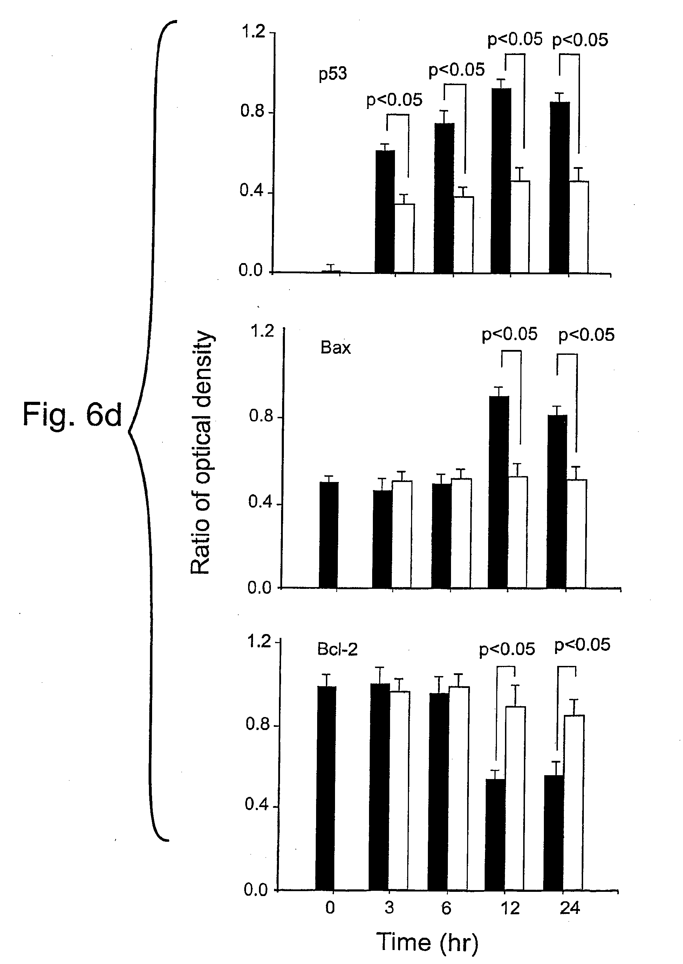

Figures 6a-6f show that APC blocks p53-dependent apoptosis in NMDA-treated mouse cortical neurons. Figs. 6a-6b, Western blot analyses for p53 in nuclear protein extracts from NMDA-treated cells in the absence or presence of human APC (100 nM) at different time points are illustrated. Fig. 6c, Western blots analyses for Bax and Bcl-2 on whole cell extracts from experiments similar to those shown in Fig. 6a are illustrated. Fig. 6d, Densitometric analyses of optical density of p53, Bax, and Bcl-2 bands normalized to β-acting are shown for NMDA-treated cells in the absence (open) or presence (filled) of human APC. Data are mean ± SEM (3 to 5 independent measurements for each time point). Fig. 6e, Electrophoretic mobility shift assays show no change in NF-κB DNA binding activities following exposure of neurons to NMDA. Human umbilical vein endothelial cells (HUVEC) exposed to *E. coli* LPS served as a positive control. Fig. 6f, Western blot analyses for intact cortical NMDA receptor subunits NR1 and NR2A in membrane fractions 24 hr after treatment with human APC (100 nM) are illustrated.

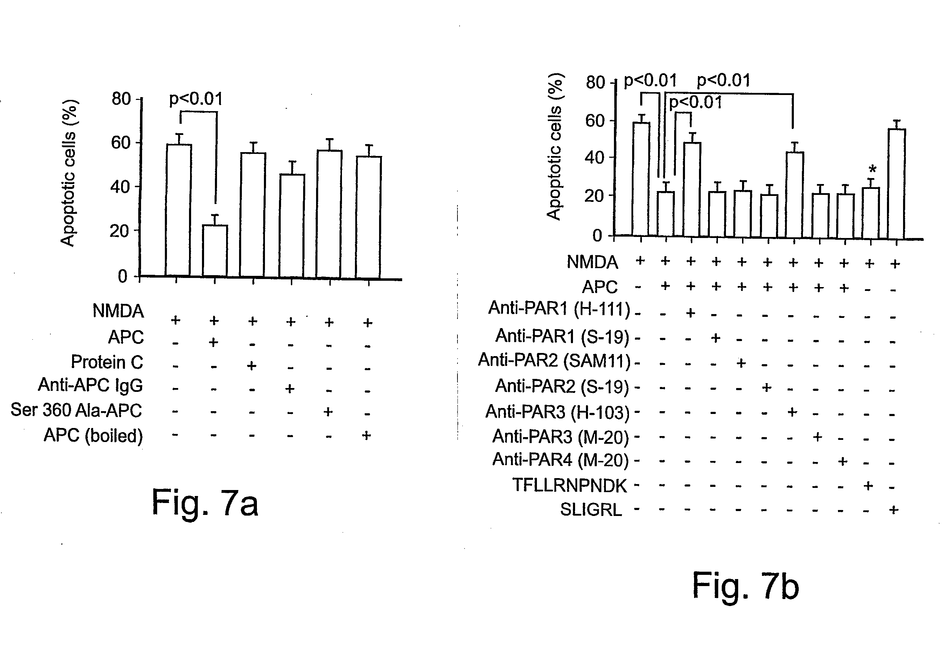

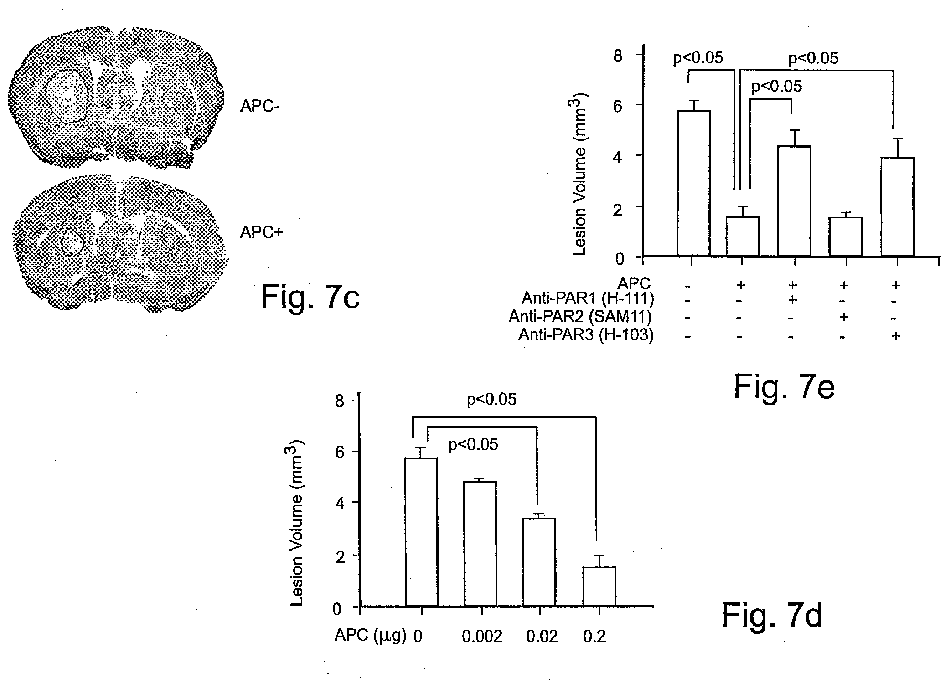

Figures 7a-7e show the specificity of APC protection on NMDA-induced neuronal death in cultured mouse cortical neurons and in mouse brains *in vivo.* Fig. 7a, Cortical neurons were treated with NMDA and incubated for 24 hr with vehicle, human APC (100 nM), protein C zymogen (100 nM), anti-APC IgG (C3), Ser360Ala-APC (100 nM) and boiled APC (100 nM); apoptosis was quantitated as in Fig. 5a. Fig. 7b, Cortical neurons were treated with NMDA and incubated for 24 hr with mouse APC (10 nM) in the presence of cleavage site blocking anti-PAR-1 (H-111), anti-PAR-2 (SAM 11) or anti-PAR-3 (H-1 03) antibodies; N-terminal anti-PAR-1 (S-19) or anti-PAR-2 (S-19) antibodies and C-terminal anti-PAR-3 (M-20) or anti-PAR-4 (M-20) antibodies served as negative controls. PAR-1 agonist peptide TFLLRNPNDK (10 µM) and PAR-2 agonist peptide SLIGRL (100 µM) were also studied. The percentage of apoptotic cells was quantitated as in Fig. 7a. Data are mean ± SEM (n = 3-5 independent measurements in triplicate) for Figs. 7a-7b. *P < 0.01, NMDA vs. NMDA + TFLLRNPNDKAPC. Fig. 7c, Coronal sections of mouse brains infused with NMDA in the absence (APC-) or presence (APC+) of mouse APC (0.2 µg) were stained with cresyl violet. Fig. 7d, Dose-dependent protective effect of mouse APC on NMDA-induced injury (lesion volume) in mouse striatum was determined at 48 hr. Fig. 7e, Effects of different anti-PAR antibodies on NMDA lesion volumes in the absence or presence of mouse APC: NMDA + APC (0.2 µg); NMDA + APC (0.2 µg) + anti-PAR-1 (H-111, 0.2 µg), anti-PAR-2 (SAM11, 0.2 µg), or anti-PAR-3 (H-103, 0.2 µg) are shown. Data are mean ± SEM, n = 3-5 mice for Figs. 7d-7e.

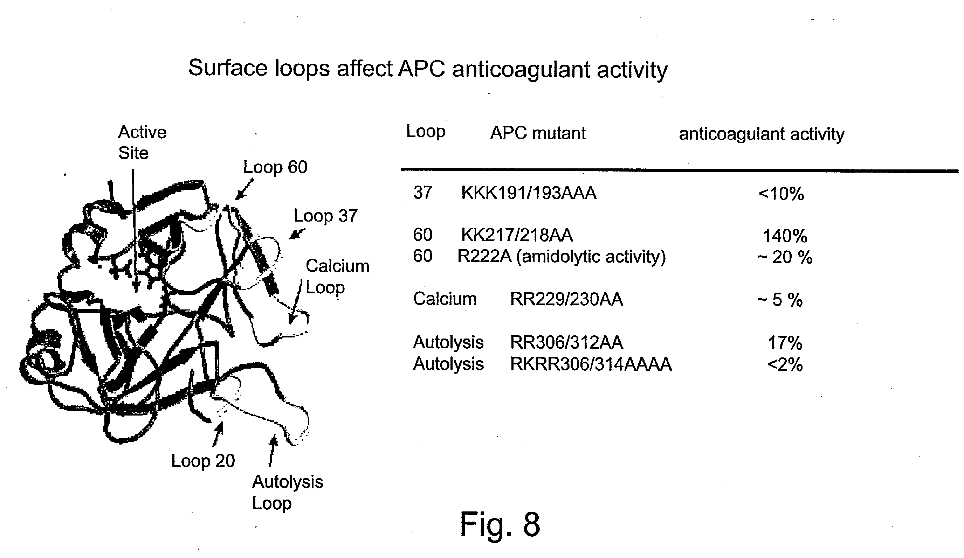

Figure 8 illustrates surface loops in the vicinity of the protease active site of APC with the numbering scheme of chymotrypsin indicated. Anticoagulant activity of human APC mutants (Gale et al., Blood 96:585-593, 2000; Gale et al., J. Biol. Chem. 277:28836-28840, 2002) is expressed as a percentage of recombinant wild-type human APC (defined as 100%).

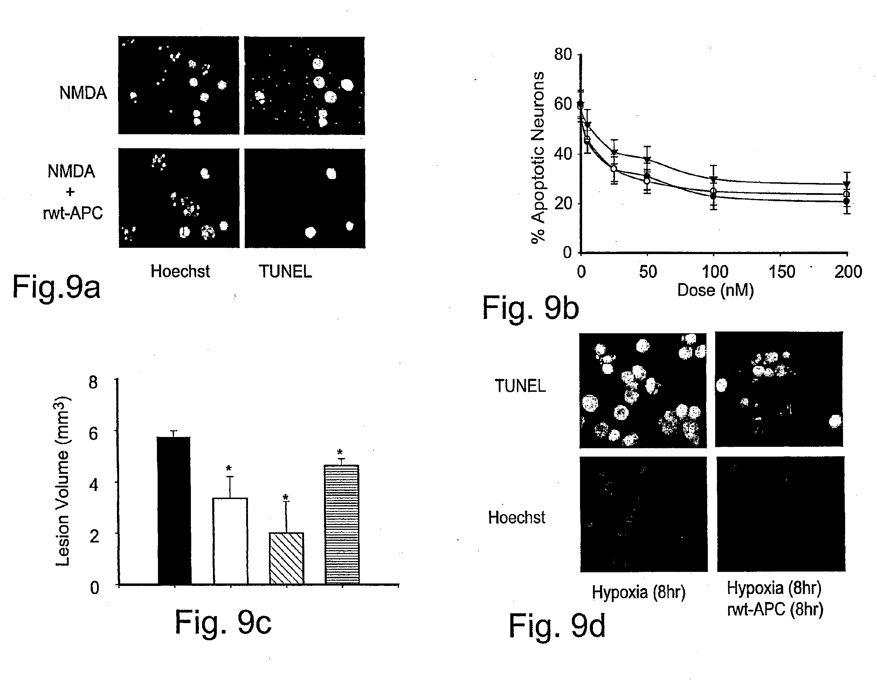

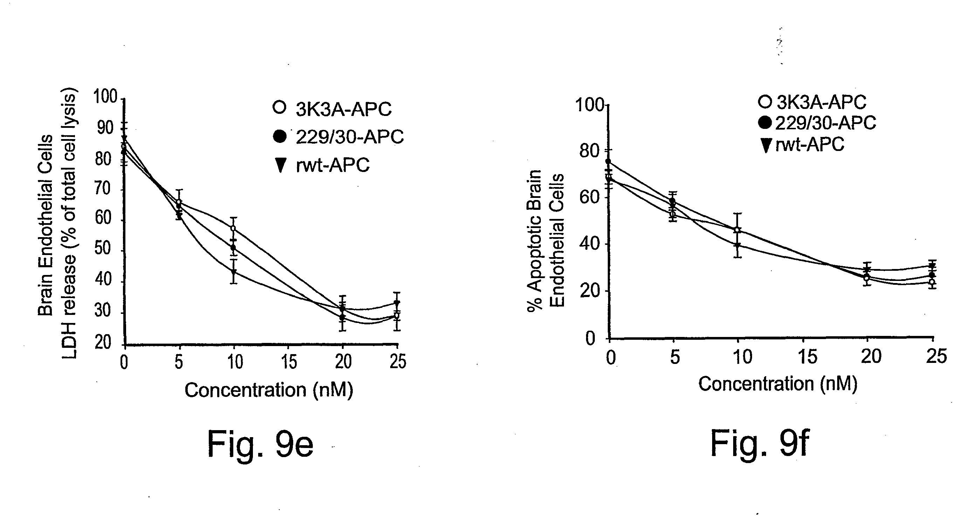

Figures 9a-9f show the direct neuroprotective effects of human APC mutants in either loop 37 (KKK191-193AAA, "3K3A-APC") or the Ca⁺⁺-binding loop (RR229/230AA, "229/30-APC") as compared to recombinant wild-type human APC ("rwt-APC"). Fig. 9a, Immunostaining for TUNEL and Hoechst in isolated mouse neurons 24 hr after exposure to 300 µM NMDA in the absence or presence of rwt-APC (100 nM). Fig. 9b, Dose-dependent neuroprotective effects of APC mutants and rwt-APC on isolated neurons at 24 hr of exposure to 300 µM NMDA. Filled circle, rwt-APC; open circle, 3K3A-APC; triangle, 229/30APC. Fig. 9c, Lesion volume in mouse brain infused with NMDA (20 nmol) in the absence or presence of APC mutants or rwt-APC (0.2 µg). Filled bar, NMDA only; open bar, NMDA and rwt-APC; diagonally hashed bar, NMDA and 3K3A-APC; horizontally hashed bar, NMDA and 229/30APC. Fig. 9d, Immunostaining for TUNEL and Hoechst of isolated human brain endothelial cells (BEC) 8 hr after hypoxia/aglycemia in the absence or presence of rwt-APC (100 nM). Figs. 9e-9f, Dose-dependent neuroprotective effects of APC mutants and rwt-APC on hypoxic human BEC at 8 hr of hypoxia/aglycemia estimated from LDH release and the percentage of apoptotic BEC by TUNEL. Open circle, 3K3A-APC; filled circle, 229/30APC; triangle, rwt-APC. Data are mean ± SEM (3-5 independent measurements).

### DESCRIPTION OF SPECIFIC EMBODIMENTS OF THE INVENTION

The present invention is useful for treating neurodegenerative diseases involving apoptosis and/or cell death in the central nervous system as defined in the claims. Inhibition of p53 signaling by activated protein C (APC) or a variant thereof may be demonstrated by *in vitro* and *in vivo* assays (e.g., cell cultures and animal models). Apoptosis and/or cell death are reduced as a result of practicing the invention. Injury due to ischemia or hypoxia may be prevented or at least mitigated. Similarly, injury from ultraviolet (UV) or gamma irradiation (i.e., physical insults of the environment) or chemical contaminants and pollutants may be prevented or at least mitigated. In particular, neurotoxicity due to overstimulation of N-methyl-D-aspartate (NMDA) receptors is a useful model for neuronal cell injury and death that mimics the effects of neurodegenerative disease. Cytoprotection may be determined at the level of different cell types, organs or tissues, or whole organisms.

The present invention provides methods for protecting neuronal cells from cell death in a subject having or at risk of neurodegenerative disease as defined in the claims. The method includes administering an effective amount of activated protein C, a prodrug, or a variant thereof to the subject wherein the functional variant is comprised of at least the mutation KKK191-193AAA, thereby providing neuroprotection to the subject. In certain embodiments, the effective amount may be a low dose of APC or the variant thereof which is directly neuroprotective but with at least reduced anticoagulant activity as compared to prior art treatments. Variants of APC with reduced anticoagulant activity have been described (Gale et al., J. Biol. Chem. 277:28836-28840, 2002). Examples of such diseases include, ischemia and stroke. Improvement in treating neurodegenerative disease may be clinically measurable by neurological or psychiatric tests; similarly, therapeutic effects on coagulation, fibrinolysis, thrombosis, and inflammation may be clinically determined.

In neurodegenerative diseases, neuronal cells degenerate to bring about deterioration of cognitive function. A variety of diseases and neurological deficiencies may bring about such degeneration including Alzheimer's disease, Huntington disease or chorea, hypoxia or ischemia caused by stroke, cell death caused by epilepsy, amyotrophic lateral sclerosis, mental retardation and the like, as well as neurodegenerative changes resulting from aging.

The term "neurodegenerative disease" is used to denote conditions which result from loss of neurons, neuronal cell injury or loss, and/or injury of other types of brain cells such as oligodendrocytes, brain endothelial cells, other vascular cells, and/or other cell types in the nervous system which may bring about deterioration of a motor or sensory function of the nervous system, cognitive function, higher integrative intellectual functions, memory, vision, hearing etc. Such degeneration of neural cells may be caused pathological conditions caused by temporary lack of blood or oxygen supply to the brain, e.g. brought about by stroke. It should be noted that diseases such as stroke has both a neurodegenerative and a vascular component, with or without an inflammatory response, and thus can be treated by the methods of the invention.

Disclosed are activated protein C's activities such as a inhibitor of apoptosis or cell death, cell survival factor, and cytoprotective agent. The cell may be derived from brain vessels (e.g., an endothelial or smooth muscle cell) of a subject, especially from the endothelium of a brain vessel. Alternatively, it may be a neuron, an astrocyte, a microglial cell, an oligodendrocyte, or a pericyte; a precursor or a progenitor cell thereof; or other types of differentiated cell from the subject's central or peripheral nervous system.

In particular, "neuron" includes hundreds of different types of neurons, each with distinct properties. Each type of neuron produces and responds to different combinations of neurotransmitters and neurotrophic factors. Neurons are thought not to divide in the adult brain, nor do they generally survive long *in vitro.* The method of the disclosure provides for the protection from death or senescence of neurons from virtually any region of the brain and spinal cord. Neurons include those in embryonic, fetal or adult neural tissue, including tissue from the hippocampus, cerebellum, spinal cord, cortex (e.g., motor or somatosensory cortex), striatum, basal forebrain (e.g., cholinergic neurons), ventral mesencephalon (e.g., cells of the substantia nigra), and the locus ceruleus (e.g., neuroadrenaline cells of the central nervous system).

There are many other diseases which benefit from the methodologies of the present disclosure such as for example, coronary arterial occlusion, cardiac arrhythmias, congestive heart failure, cardiomyopathy, bronchitis, neurotrauma, graft/transplant rejection, myocarditis, diabetic neuropathy, and stroke. Life threatening local and remote tissue damage occurs during surgery, trauma, and stroke when major vascular beds are deprived for a time of oxygenation (ischemia) then restored with normal circulation (reperfusion). Cell death and tissue damage can lead to organ failure or decreased organ function. The compositions and methodologies of the present invention are useful in treatment of ischemia and stroke.

"Protein C" refers to native genes and proteins belonging to this family as well as variants thereof (e.g., mutations and polymorphisms found in nature or artificially designed). The chemical structure of the genes and proteins may be a polymer of natural or non-natural nucleotides connected by natural or non-natural covalent linkages (i.e., polynucleotide) or a polymer of natural or non-natural amino acids connected by natural or non-natural covalent linkages (i.e., polypeptide). See Tables 1-4 of WIPO Standard ST.25 (1998) for a nonlimiting list of natural and non-natural nucleotides and amino acids. Protein C genes and proteins may be recognized as belonging to this family by comparison to the human homolog PROC, use of nucleic acid binding (e.g., stringent hybridization under conditions of 400 mM NaCl, 40 mM PIPES pH 6.4, 1 mM EDTA, at 50°C or 70°C for an oligonucleotide; 500 mM NaHPO₄ PH 7.2, 7% SDS, 1% BSA, 1 mM EDTA, at 45°C or 65°C for a polynucleotide of 50 bases or longer; and appropriate washing) or protein binding (e.g., specific immunoassay under stringent binding conditions of 50 mM Tris-HCl pH 7.4, 500 mM NaCl, 0.05% TWEEN 20 surfactant, 1% BSA, at room temperature and appropriate washing); or computer algorithms (Doolittle, Of URFS and ORFS, 1986; Gribskov & Devereux, Sequence Analysis Primer, 1991; and references cited therein).

A "mutation" refers to one or more changes in the sequence of polynucleotides and polypeptides as compared to native protein C, and has at least one function that is more active or less active, an existing function that is changed or absent, a novel function that is not naturally present, or combinations thereof. In contrast, a "polymorphism" also refers to a difference in its sequence as compared to native protein C, but the changes do not necessarily have functional consequences. Mutations and polymorphisms can be made by genetic engineering or chemical synthesis, but the latter is preferred for non-natural nucleotides, amino acids, or linkages. Fusions of domains linked in their reading frames are another way of generating diversity in sequence or mixing-and-matching functional domains. For example, homologous protein C and protein S work best together and this indicates that their sequences may have coevolved to optimize interactions between the enzyme and its cofactor. Exon shuffling or gene shuffling techniques may be used to select desirable phenotypes in a chosen background (e.g., separable domains with different biological activities, hybrid human/mouse sequences which locate the species determinants).

Percentage identity between a pair of sequences may be calculated by the algorithm implemented in the BESTFIT computer program (Smith & Waterman. J. Mol. Biol. 147:195-197, 1981; Pearson, Genomics 11:635-650, 1991). Another algorithm that calculates sequence divergence has been adapted for rapid database searching and implemented in the BLAST computer program (Altschul et al., Nucl. Acids Res. 25:3389-3402, 1997). In comparison to the human sequence, the protein C polynucleotide or polypeptide may be only about 60% identical at the amino acid level, 70% or more identical, 80% or more identical, 90% or more identical, 95% or more identical, 97% or more identical, or greater than 99% identical.

Conservative amino acid substitutions (e.g., Glu/Asp, Val/Ile, Ser/Thr, Arg/Lys, Gln/Asn) may also be considered when making comparisons because the chemical similarity of these pairs of amino acid residues are expected to result in functional equivalency in many cases. Amino acid substitutions that are expected to conserve the biological function of the polypeptide would conserve chemical attributes of the substituted amino acid residues such as hydrophobicity, hydrophilicity, side-chain charge, or size. In comparison to the human sequence, the protein C polypeptide may be only about 80% or more similar, 90% or more similar, 95% or more similar, 97% or more similar, 99% or more similar, or about 100% similar. Functional equivalency or conservation of biological function may be evaluated by methods for structural determination and bioassay.

The codons used may also be adapted for translation in a heterologous host by adopting the codon preferences of the host. This would accommodate the translational machinery of the heterologous host without a substantial change in chemical structure of the polypeptide.

Protein C and variants thereof (i.e., deletion, domain shuffling or duplication, insertion, substitution, or combinations thereof) may be used to determine structure-function relationships (e.g., alanine scanning, conservative or nonconservative amino acid substitution). For example, protein C folding and processing, secretion, receptor binding, signaling through EPCR, PAR-1, and/or PAR-3, inhibition of p53 signaling, any of the other biological activities described herein, or combinations thereof may be related to changes in the amino acid sequence. See Wells (Bio/Technology 13:647-651, 1995) and U.S. Patent 5,534,617. Directed evolution by directed or random mutagenesis or gene shuffling using protein C may be used to acquire new and improved functions in accordance with selection criteria. Mutant and polymorphic variant polypeptides are encoded by suitable mutant and polymorphic variant polynucleotides. Structure-activity relationships of protein C may be studied (i.e., SAR studies) using variant polypeptides produced with an expression construct transfected in a host cell with or without expressing endogenous protein C. Thus, mutations in discrete domains of protein C may be associated with decreasing or even increasing activity in the protein's function.

Gale et al. (J. Biol. Chem. 277:28836-28840, 2002) have demonstrated that mutations in the surface loops of APC affect its anticoagualant activity. It has been shown that APC mutants KKK191/193AAA (loop 37), RR229/230AA (calcium loop), RR306/312AA (autolysis loop), RKRR306/314AAAA (autolysis loop) have approximately < 10%, 5%, 17%, and < 2% of the anticoagulant activity of native APC, respectively. A follow-up study (Mosnier, Gale, & Griffin, unpublished observations) has demonstrated that these APC mutants with reduced anticoagulant activity (i.e., KKK191/193AAA, RR229/230AA, RR306/312AA and RKRR306/312AAAA) retain the anti-apoptotic activity of APC in staurosporine model of apoptosis.

Protein C zymogen, the precursor of activated protein C, is readily converted to activated protein C within the body by proteases. Protein C may be considered a prodrug form of activated protein C. Thus, the use of activated protein C is expressly intended to include protein C and variants thereof. Treatments with protein C would require appropriately larger doses known to those of skill in the art (see below).

Recombinant forms of protein C can be produced with a selected chemical structure (e.g., native, mutant, or polymorphic). As an illustration, a gene encoding human protein C is described in U.S. Patent 4,775,624 and can be used to produce recombinant human protein C as described in U.S. Patent 4,981,952. Human protein C can be recombinantly produced in tissue culture and activated as described in U.S. Patent 6,037,322. Natural human protein C can be purified from plasma, activated, and assayed as described in U.S. Patent 5,084,274. The nucleotide and amino acid sequence disclosed in these patents may be used as a reference for protein C.

Dosages, dosing protocols, and protein C variants that reduce bleeding in a subject as compared to activated protein C which is endogenous to subject are preferred. Mutations in the sequence of native protein C may separate the ability to reduce p53 signaling from other biological activities (e.g., anticoagulant activity). The cytoprotective activity of protein C may thereby be maintained or increased while decreasing undesirable side effects of the administration of activated protein C (e.g., bleeding in the brain and other organs).

### FORMULATIONS AND THEIR ADMINISTRATION

Pharmaceutical compositions as defined in the claims may be administered *in vitro* to cells in culture, *in vivo* to cells in the body, or *ex vivo* to cells outside of a subject which may then be returned to the body of the same subject or another. The cells may be removed from, transplanted into, or be present in the subject (e.g., genetic modification of endothelial cells *in vitro* and then returning those cells to brain endothelium). The cell may be from the endothelium (e.g., an endothelial or smooth muscle cell), especially from the endothelium of a brain vessel. It may also be a neuron; a glial cell; a precursor, progenitor, or stem cell thereof; or another differentiated cell from the central or peripheral nervous system.

Use of compositions which further comprise a pharmaceutically acceptable carrier and compositions which further comprise components useful for delivering the composition to a subject's brain are known in the art. Addition of such carriers and other components to the composition of the invention is well within the level of skill in this art. For example, a permeable material may release its contents to the local area or a tube may direct the contents of a reservoir to a distant location of the brain.

A pharmaceutical composition may be administered as a formulation which is adapted for direct application to the central nervous system, or suitable for passage through the gut or blood circulation. Alternatively, pharmaceutical compositions may be added to the culture medium. In addition to active compound, such compositions may contain pharmaceutically-acceptable carriers and other ingredients known to facilitate administration and/or enhance uptake. It may be administered in a single dose or in multiple doses which are administered at different times. A unit dose of the composition is an amount of APC or APC mutant comprised of at least the mutation KKK191-193AAA which provides neuroprotection, cytoprotection, inhibits apoptosis or cell death, and/or promotes cell survival but does not provide a clinically significant anticoagulant, profibrinolytic, or antithrombotic effect, a therapeutic level of such activity, or has at least reduced activity in comparison to previously described doses of activated protein C. Measurement of such values are within the skill in the art: clinical laboratories routinely determine these values with standard assays and hematologists classify them as normal or abnormal depending on the situation.

Pharmaceutical compositions may be administered by any known route. By way of example, the composition may be administered by a mucosal, pulmonary, topical, or other localized or systemic route (e.g., enteral and parenteral). In particular, achieving an effective amount of activated protein C, prodrug, or functional variant in the central nervous system may be desired. This may involve a depot injection into or surgical implant within the brain. "Parenteral" includes subcutaneous, intradermal, intramuscular, intravenous, intra-arterial, intrathecal, and other injection or infusion techniques, without limitation.

Suitable choices in amounts and timing of doses, formulation, and routes of administration can be made with the goals of achieving a favorable response in the subject (i.e., efficacy), and avoiding undue toxicity or other harm thereto (i.e., safety). Thus, "effective" refers to such choices that involve routine manipulation of conditions to achieve a desired effect (e.g., inhibition of apoptosis or cell death, promotion of cell survival, neuroprotection, cytoprotection, or combinations thereof). In this manner, "effective amount" refers to the total amount of activated protein C, prodrug (e.g., protein C), or functional variant which achieves the desired effect. Activity can be determined by reference to a low amount of activated protein C (e.g., 0.005 mg/kg or less, 0.01 mg/kg or less, 0.02 mg/kg or less, 0.03 mg/kg or less, 0.04 mg/kg of less); similarly, an "equivalent amount" of prodrug or functional variant with reduced anticoagulant activity can be determined by achieving the same or similar desired neuro-protecive effect as the reference amount of activated protein C, but with reduced risk for bleeding due to reduced anticoagulant activity.

A bolus of the formulation administered only once to a subject is a convenient dosing schedule although achieving an effective concentration of activated protein C in the brain may require more frequent administration. Treatment may involve a continuous infusion (e.g., for 3 hr after stroke) or a slow infusion (e.g., for 24 hr to 72 hr when given within 6 hr of stroke). Alternatively, it may be administered every other day, once a week, or once a month. Dosage levels of active ingredients in a pharmaceutical composition can also be varied so as to achieve a transient or sustained concentration of the compound or derivative thereof in a subject and to result in the desired therapeutic response. But it is also within the skill of the art to start doses at levels lower than required to achieve the desired therapeutic effect and to gradually increase the dosage until the desired effect is achieved.

The amount of compound administered is dependent upon factors such as, for example, bioactivity and bioavailability of the compound (e.g., half-life in the body, stability, and metabolism); chemical properties of the compound (e.g., molecular weight, hydrophobicity, and solubility); route and scheduling of administration; and the like. It will also be understood that the specific dose level to be achieved for any particular subject may depend on a variety of factors, including age, health, medical history, weight, combination with one or more other drugs, and severity of disease.

For example, a low dose may be used to prevent apoptosis or cell death and/or to promote cell survival. These effects are different from APC's inhibition of thrombosis, clotting, and/or inflammation which were obtained at higher doses. APC's anti-inflammatory effects are possibly mediated by down regulation of the NF-κB pathway. In homologous systems (e.g., human native or recombinant APC administered to patients), a single bolus of APC (e.g., 0.005 mg/kg or less, 0.01 mg/kg or less, 0.02 mg/kg or less, 0.03 mg/kg or less, 0.04 mg/kg or less administered over 1 min) may be sufficient to be directly neuroprotective without having a significant anti-thrombotic effect in brain circulation. Previous treatment infused APC at a dosage of 0.01 mg/kg/hr to 0.05 mg/kg/hr for 4 hr to 96 hr. Thus, less than 0.005 mg/kg, less than 0.01 mg/kg, less than 0.02 mg/kg, less than 0.03 mg/kg, or less than 0.04 mg/kg are doses which can be formulated and administered in accordance with the teachings herein. An illustrative amount may be calculated for a 70 kg adult human, and this may be sufficient to treat humans of between 50 kg and 90 kg.

The effective or equivalent amount may be packaged in a "unit dose" with written instructions for achieving one or more desired effects and/or avoiding one or more undesired effects. The aforementioned formulations, routes of administration, and dosing schedules are merely illustrative of the techniques which may be used.

The term "treatment" refers to, inter alia, reducing or alleviating one or more symptoms of neurodegenerative disease. For example, standard therapy such as stroke treatment with a tissue plasminogen activator may be compared with and without activated protein C, a drug, or a variant thereof. This includes therapy of an affected subject or prophylaxis of a subject at risk. For a given subject, improvement in a symptom, its worsening, regression, or progression may be determined by objective or subjective measures. The subject in need of treatment may be at risk for or already affected by neurodegenerative disease; treatment may be initiated before and/or after diagnosis. In a patient, an indication that treatment is effective may be improved neurological outcome, motor or sensory functions, cognitive functions, psychomotor functions, motor neurological functions, higher integrative intellectual functions, memory, vision, hearing, etc.; reduced brain damage and injury as evidenced by noninvasive image analysis (e.g., MRI or brain perfusion imaging); or combinations thereof. This effect may be confirmed by neuropathological analysis of brain tissue. Ultimately, reduction in a neurodegenerative process by stabilizing brain endothelial cell functions and preventing their death will lead to improvements in the cerebral blood flow (CBF) and normalization of CBF regulatory functions. In a pre-clinical study, neurological or behavioral findings, reduction in apoptosis or a marker thereof (e.g., DNA content and fragmentation), increased cell survival, decreased cell death, or combinations thereof can be demonstrated in an animal model. These benefits may be achieved with little or no significant system anticoagulation in human or animal subjects. At the cellular level, reduced p53 signaling, normalized Bax/Bcl-2 ratio, reduced caspase-3 signaling, or combinations thereof may be observed. Increase or decrease may be determined by comparison to treatment with or without activated protein C, a prodrug, or a variant thereof, or to the expected effects of untreated disease.

Treatment may also involve other existing modes of treatment and agents (e.g., protein S, fibrinolytic or antithrombotic agents, steroidal or nonsteroidal anti-inflammatory agents). Thus, combination treatment may be practiced (e.g., APC and tPA administered concurrently or sequentially).

### EXAMPLES

APC, a systemic anticoagulant and anti-inflammatory factor¹⁻³, reduces organ damage in animal models of sepsis, ischemic injury and stroke^{1,4,5}. APC significantly reduces mortality in patients with severe sepsis⁶. Whether APC acts as a direct cell survival factor or whether the neuroprotection by APC^{5,7} is secondary to its anticoagulant and anti-inflammatory effects is not known¹⁻³. Here, we show that APC prevents apoptosis in hypoxic human brain endothelium through transcriptionally-dependent inhibition of tumor suppressor protein p53, normalization of the Bax/Bcl-2 ratio, and reduction of caspase-3 signaling. These mechanisms are distinct from the previously shown upregulation of anti-apoptotic genes (e.g., Bcl2-related protein A1, inhibitor of apoptosis 1) by APC in human umbilical vein endothelial cells^{8,9}. APC's cytoprotection of brain endothelium *in vitro* required endothelial protein C receptor (EPCR) and protease activated receptor 1 (PAR-1), as did APC's *in vivo* neuroprotective activity in an ischemic stroke model⁵ in mice with a severe deficiency of EPCR¹⁰, consistent with work showing APC direct effects on cultured cells via EPCR and PAR-1⁹. Moreover, the *in vivo* neuroprotective effects of low dose APC appeared to be independent of its anticoagulant activity. Thus, APC protects brain from ischemia by acting directly on brain cells.

Moreover, we show that APC can directly protect perturbed neurons from cell injury and apoptosis. We report that APC interferes with N-methyl-D-aspartate (NMDA) apoptosis in cultured mouse cortical neurons by blocking p53 and caspase-3 pro-apoptotic signaling. Direct intracerebral infusions of APC significantly reduced NMDA excitotoxic brain lesions in mice suggesting that APC's systemic effects are not required for brain protection. APC's direct neuroprotective effects on perturbed mouse neurons *in vitro* and *in vivo* required PAR-1 and PAR-3 on neurons, consistent with our findings in hypoxic brain endothelial cells that EPCR-dependent signaling by APC through PAR-1 prevents p53-dependent apoptosis of endothelium. Thus, the present work also demonstrates that APC directly prevents *in vitro* and *in vivo* NMDA-induced neuronal apoptosis, suggesting APC may limit neuronal damage in neurodegenerative disorders caused by overstimulation of NMDA receptors.

### EXAMPLE 1:

*Reagents and antibodies.* Human APC, protein C zymogen, mutant Ser360Ala-APC lacking the active site serine, recombinant murine APC, and mouse anti-human APC IgG (C3 antibody) were prepared as described^{17,24} or using known techniques. For Western blots and immunostaining, we used the following antibodies: p53, mouse anti-human monoclonal, 1:100 (0.4 mg/ml, DAKO); Bcl-2, mouse anti-human monoclonal, 1:100 (0.2 mg/ml, Santa Cruz Biologics); Bax, mouse anti-human monoclonal, 1:100 (0.2 mg/ml, Santa Cruz Biologics); Bcl2-related protein A1 or Bcl2A1, rabbit anti-human polyclonal 1:100 (0.2 mg/ml, Santa Cruz Biologics); inhibitor of apoptosis 1 or clAP1, rabbit anti-human polyclonal 1:100 (0.2 mg/ml, Santa Cruz Biologics); endothelial nitric oxide synthase or eNOS, rabbit anti-human polyclonal 1:100 (0.2 mg/ml, Santa Cruz Biologics); β-actin, goat anti-human polyclonal, 1:2500 (0.2 mg/ml, Santa Cruz Biologics); active caspase-3, rabbit anti-human, 1:250 (1 mg/ml, Promega); Von Willebrand Factor, rabbit anti-human monoclonal, 1:200 (5.6 mg/ml, DAKO); GFAP, glial fibrillar acidic protein, mouse anti-bovine polyclonal, 1:500 (11.7 mg/ml, DAKO); and CD11 b, mouse anti-human monoclonal, 1:100 (0.2 mg/ml, Oncogene). Antibodies blocking activation of PAR-1 (H-111), PAR-2 (SAM-11), or PAR-3 (H-1 03) were obtained from Santa Cruz Biologics. Anti-EPCR antibodies that block (RCR-252) or do not block (RCR-92) APC binding have been described¹⁸. PAR-1 and PAR-2 agonist peptides TFLLRNPNDK and SLIGRL, respectively, were obtained from Ana Spec (San Jose, CA). Hirudin was from Sigma (St. Louis, MO).

*Human microvascular brain endothelial cells (BEC).* Primary BEC were isolated from rapid (less than 3 hr) autopsies from neurologically normal young individuals after trauma. BEC were characterized and cultured as described previously²⁷. After FACS sorting using Dil-Ac-LDL, cells were greater than 98% positive for the endothelial markers von Willebrand factor and CD105, and negative for GFAP (astrocytes), CD11 b (macrophages/microglia) and α-actin (smooth muscle cells). Early passage (P3-P5) cells were used for all studies.

*Hypoxia model.* We used hypoxia/aglycemia as an *in vitro* model of ischemic injury as described¹⁴. Briefly, 0.7 x 10⁶ BEC were seeded on 100 mm plate in RPMI1640 medium supplemented with 20% fetal bovine serum, endothelial cell growth supply (30 µg/ml, Sigma), and heparin (5 U/ml, Sigma). Twenty-four hours later, the cells were washed twice with PBS and then transferred to serum-free Dulbecco's Modified Eagle Media (DMEM) medium without glucose and exposed to severe hypoxia (less than 2% oxygen) using an anaerobic chamber (Forma Scientific) equipped with a humidified, temperature-controlled incubator. The entire system was purged with 95% N₂/5% CO₂ atmosphere. The oxygen levels in the incubator were monitored by O₂ Fyrite (Forma Scientific). Control BEC were maintained in DMEM supplemented with 20% oxygen and 5 mM glucose. In most studies, experiments were performed at 8 hr of culture when the hypoxic injury was already maximal.

*Detection of cell injury and apoptosis.* Cell injury was initially detected by the release of lactate dehydrogenase (LDH) into the cell culture medium using an LDH assay (Sigma) according to manufacturer's instructions. The Hoechst and TUNEL staining were performed to determine the apoptosis on acetone fixed cells. For the Hoechst staining, cells were stained for 5 min with 1 µg/ml of the fluorescent DNA-binding dye Hoechst 33,342 (Sigma). The TUNEL assay was carried out according to the manufacturer's instructions (Phoenix Flow Systems). The images were observed using an Olympus AX70 microscope.

*Western blot analysis.* Protein samples were collected for Western blot analysis after 2 hr, 4 hr, 8 hr or 24 hr of either hypoxia or normoxia (control). Protein concentration was determined using bicinchoninic acid kit (Pierce). Equal amounts of protein (10 µg/lane) were separated by 10% SDS-polyacrylamide gel electrophoresis (PAGE) and transferred onto nitrocellulose membrane. The membranes were probed with different antibodies using standard immunoblotting techniques. The relative abundance of each protein was determined by scanning densitometry using β-actin as an internal control. Comparisons between different treatments were performed within the linear intensity range of their respective signals.

*Immunofluorescence analysis.* BEC (5.6 x 10⁴/well, 12-well plate) were cultured on collagen-coated cover slips. After each treatment, cells were fixed with acetone and incubated with primary antibody at 4°C overnight followed by rhodamine-conjugated rabbit IgG (for active caspase-3 staining). The images were then examined using an Olympus AX70 microscope.

*Reverse transcription (RT)-polymerase chain reaction (PCR) analysis.* Total RNA was isolated from BEC using RNeasy Mini Kit (Qiagen). About 1 µg total RNA was used for the cDNA syntheses with SuperScript^{™} First-Strand Synthesis System (Invitrogen). Semiquantitative RT-PCR was conducted with 3 µl RT product; the thermal cycle conditions were 94°C for 4 min; 94°C for 30 sec/60°C for 30 sec/72°C for 45 sec (30 cycles); 72°C for 10 min. p53 and GAPDH specific primer sets were purchased from Biosource International. The PCR products (p53: 213 bp; GAPDH: 231 bp) were electrophoresed on 1.5% agarose gel and detected with ethidium bromide staining. Comparisons between different treatments were performed by scanning densitometry using 30 cycles that was within the linear range of the signal.

*In vivo animal model of stroke.* A modified intravascular MCAO technique⁵ was used to induce stroke. Murine recombinant APC or vehicle were administered 10 min after the MCAO via the femoral vein. CBF was monitored by laser Doppler flowmetry (Transonic Systems) as described⁵. The procedure was considered successful if a greater than or equal to 80% drop in CBF was observed immediately after placement of the suture. Arterial blood gasses were measured before and during MCAO as described⁵. Neurological studies were performed at 24 hr and animals were sacrificed at that time for neuropathological analysis. All animals survived 24 hr. Neurological examinations were scored as follows: no neurological deficit (0), failure to extend left forepaw fully (1), turning to left (2), circling to left (3), unable to walk spontaneously (4), and stroke-related death (5). Unfixed 1-mm coronal brain slices were incubated in 2% triphenyltetrazolium chloride in phosphate buffer (pH 7.4) and serial coronal sections were displayed on a digitizing video screen (Jandel Scientific). Brain infarct and edema volume were calculated with Swanson correction as described⁵. The effect of murine APC (0.2 mg/kg) was determined in mice with severe deficiency in EPCR¹⁰ and in wild-type C57BL/6 mice in the presence and absence of anti-PAR-1 antibodies (H-111, 40 µg/mouse) infused 10 min prior the MCAO. In a separate studies, the effects of low dose murine APC (0.04 mg/kg) and high dose murine APC (0.2 mg/kg) were compared. In these experiments, fibrin was quantified by Western blotting with anti-fibrin II antibody (NYB-T2G1, Accurate Chemical Scientific Corp.) and leukocytes were stained with CD11 b antibody (DAKO, 1:250)⁵.

*Statistics.* Data were presented as mean ± SD. ANOVA was used to determine statistically significant differences; non-parametric data (neurological outcome scores) were compared by the Kruskal-Wallis test. *P* < 0.05 was considered statistically significant.

Endothelial dysfunction is critical during ischemic injury including ischemic brain damage. Stress signals¹¹⁻¹³ and hypoxia¹⁴⁻¹⁶ cause cellular injury partly through the action of the tumor suppressor protein p53. To test the hypothesis that APC exerts anti-apoptotic effects during ischemic brain damage by preventing p53-mediated apoptosis, we used a model of hypoxic brain endothelial cell (BEC) injury¹⁴. Fig. 1a illustrates time-dependent release of lactate dehydrogenase (LDH) from hypoxic primary human BEC. LDH release (corrected for the basal release under normoxic conditions) indicated hypoxic injury in 60% to 70% of cells between 8 hr and 24 hr. APC exerted a dose-dependent cytoprotective effect (Fig. 1 b) and prevented hypoxic injury in 55% to 65% of cells. Hirudin, a specific thrombin inhibitor (1 µg/ml), did not have an effect on APC protective activity. Normoxic cells were rarely TUNEL-positive (Fig. 1c, upper left panel; Fig. 1 d). In contrast, hypoxic BEC were greater than 65% TUNEL-positive and also exhibited chromatin condensation and nuclear shrinkage (Fig. 1c, middle panels; Fig. 1d). In the presence of APC and hypoxia, the number of TUNEL-positive cells and cells with apoptotic nuclear changes were significantly reduced by up to 60% (Fig. 1c, lower panels; Fig. 1d). Fig. 1d demonstrates dose-dependent anti-apoptotic effect of APC in hypoxic BEC with EC₅₀ of about 15 nM. These studies were performed with human APC administered to human cells.

To define the specificity for APC protective effects, the effects of hypoxia/ ischemia on BEC was examined in the presence of recombinant mutant Ser360Ala-APC lacking the active site serine¹⁷, protein C zymogen, heat inactivated APC, and APC plus the neutralizing C3 anti-APC monoclonal antibody. Fig. 1e confirmed that only APC conferred direct cytoprotection, while mutant APC or protein C zymogen were not protective. This suggests that the active site serine is necessary for the cytoprotective APC effects, raising a possibility that protease activated receptor (PAR) receptors may be involved.

Therefore, we studied next the effects of APC in the presence of cleavage blocking antibodies against PAR-1, PAR-2 and PAR-3 whose mRNA transcripts were present in human BEC as confirmed by the microarray gene expression analysis. Blockage of the PAR-1 cleavage site, but not of PAR-2, resulted in a loss of APC-mediated cytoprotection (Fig. 1 e). Neutralizing antibody specific for the N-terminal cleavage-independent domain of PAR-1 or antibody against the PAR-3 cleavage site were without effect. An antibody against the APC binding site on endothelial protein C receptor (EPCR)¹⁸ blocked APC-mediated cytoprotection, in contrast to a control anti-EPCR antibody which does not inhibit APC binding and which had no effect (Fig. 1e). The PAR-1 agonist peptide TFLLRNPNDK, but not the PAR-2 agonist peptide SLIGRL, caused cytoprotective effects (Fig. 1e), and blocking the PAR-1 cleavage site by antibodies did not result in loss of PAR-1 agonist peptide-mediated cytoprotection. These results support the hypothesis that APC binding to EPCR and activation of PAR-1 are required to rescue brain endothelium from hypoxia-induced apoptosis. These findings are consistent with recently demonstrated APC-mediated activation of mitogen activated protein kinases in human umbilical vein endothelial cells via EPCR and PAR-1⁹.

We next determined how APC interferes with the molecular cascade involved in apoptosis in hypoxic BEC. First we demonstrated an increase in total p53 levels in hypoxic BEC by 2.1-fold and 1.5-fold at 2 hr and 4 hr, respectively (Figs. 2a-2b). Increases in Bax, a pro-apoptotic member of the Bcl-2 gene family and a transcriptional product of p53 action¹⁹, paralleled those of p53, and Bax remained elevated throughout the 24 hr (Figs. 2a-2b). Hypoxia suppressed the Bcl-2 protein, an inhibitor of apoptosis²⁰, to 30-40% of the control values (Figs. 2a-2b). These results indicated that the pro-apoptotic transcription factor p53 and an increased pro-apoptotic Bax/Bcl-2 ratio are involved in ischemic injury of brain endothelium as previously reported for hypoxic injury in several different cell types¹⁴⁻¹⁶. This chain of events resulted in activation of caspase-3, a major protease that plays a role in disassembling the nucleus by proteolysis of several nuclear substrates²¹, in about 65% of cells (Fig. 2e, middle panel, left). Caspase-3 positive cells had condensed chromatin and/or nuclear fragmentation on Hoechst staining consistent with apoptosis (Fig. 2e, middle panel, right) and increased caspase-3 activity.

APC reduced the increased levels of p53 in hypoxic BEC at 2 hr and 4 hr by 77% and 79% and of Bax by 65% and 68%, respectively (Figs. 2c-2d). In contrast, APC markedly increased the amount of Bcl-2 at 2 hr and 4 hr of hypoxia compared to vehicle-treated controls (Fig. 2c-2d). As one would predict, the APC-induced normalizations of p53 levels and of the Bax/Bcl-2 ratio were associated with a significant reduction (greater than 60%) of caspase-3 positive cells in the presence of APC (Fig. 2e, lower panel). Similar reductions in the number of cells exhibiting apoptotic nuclear changes were also observed in the presence of APC.

With respect to the intracellular mechanism of APC-induced inhibition of p53 in hypoxic BEC, we evaluated several processes known to influence the stability and proteolytic clearance of p53 including transcription and/or translation, proteolytic degradation and/or post-translational modifications¹¹⁻¹³. Hypoxia induced a rapid transient increase in p53 mRNA transcripts within the first 30 min that was strongly inhibited by APC (Figs. 3a-3b). At later time points, hypoxia did not alter the levels of p53 transcripts and they normalized within 1 hr (Fig. 3). In contrast to hypoxic BEC, APC did not affect p53 levels in normoxic cells. A major pathway regulating post-translational p53 proteosomal degradation involves binding to the oncoprotein, murine double minute-2 (Mdm2), while phosphorylation of p53 stabilizes the protein by precluding the Mdm2 binding and subsequent proteosomal degradation of p53^{11,13}. In the present model of hypoxia, p53 phosphorylation on Ser20 or Ser15, a known phosphorylation site for an ataxia telengiectasia mutated kinase in response to DNA damage¹², was undetectable. Changes in Mdm2 protein during hypoxia were undetectable, and direct effects of APC on Mdm2 protein levels were not observed.

To determine whether other anti-apoptotic genes that are upregulated by APC in HUVEC^{8,9} act synergistically with the p53 pathway in human brain endothelial cell protection, we studied protein expression of Bcl2-related protein A1 (Bcl2A1), inhibitor of apoptosis protein 1 (clAP1), and endothelial nitric oxide synthase (eNOS). Neither hypoxia nor APC significantly altered Bcl2A1 levels (Fig. 3c) consistent with the concept that Bcl2A1 does not have a marked effect on the susceptibility of HUVEC to undergo apoptosis in response to staurosporine or other apoptotic stimuli²². APC did not affect clAP1 (Fig. 3c) or eNOS levels that were increased in hypoxia at 4 hr as reported²³. These *in vitro* results imply that APC's anti-apoptotic pathway in the setting of brain endothelial ischemia involves extracellular EPCR-dependent activation of PAR-1 which results in the intracellular inhibition of p53 and Bax down regulation with a consequent decrease in Bax/Bcl-2 ratio.

To determine whether APC's *in vivo* cytoprotection has similar requirements for EPCR and PAR-1 as shown above, we studied the effects of APC during focal ischemic stroke⁵ in mice with a severe deficiency of EPCR (less than 10% of wild type)¹⁰. Administration of murine APC (0.2 mg/kg) to mice with severe EPCR deficiency and genetically-matched controls reduced brain infarction volumes by 32% and 56% (Fig. 4a), respectively, and brain edema by 45% and 73% (Fig. 4b), respectively, compared to vehicle treated controls. Thus, approximately half of the neuroprotective effect observed for 0.2 mg/kg APC was lost in mice with a severe deficiency of the EPCR suggesting an important *in vivo* role of EPCR in mediating APC's neuroprotective effects. To show the role of PAR-1 *in vivo,* 10 min before establishing the middle cerebral artery occlusion (MCAO) we infused mice with an anti-PAR-1 cleavage blocking antibody that cross reacts with murine PAR-1, and at 10 min after the MCAO, mice received either vehicle or APC (0.2 mg/kg). There was a significant 1.5-fold and 2-fold increase in the infarction (Fig. 4c) and edema volumes (Fig. 4d), respectively, at 24 hr between mice treated with APC plus anti-PAR-1 antibody and mice treated with APC only. This indicated that, in the presence of anti-PAR-1 antibody, the ability of APC to protect mice effectively against focal ischemic insult is markedly reduced. These *in vivo* studies corroborate our mechanistic *in vitro* findings by demonstrating that two receptors essential for APC's cytoprotection of brain endothelium during ischemia *in vitro,* i.e., EPCR and PAR-1, contribute significantly to APC's neuroprotective effects *in vivo.*

To assess whether the *in vivo* mechanisms of APC involve anticoagulant and anti-inflammatory pathways as well as anti-apoptotic effects, we determined the volume of brain injury, change in the cerebral blood flow (CBF), and the deposition of cerebrovascular fibrin and neutrophils⁵ in the presence of low dose APC (0.02 or 0.04 mg/kg) or high dose APC (0.2 mg/kg) administered 10 min after the establishment of MCAO. Low dose APC significantly reduced motor neurological score (Fig. 4e) and volume of brain injury (Fig. 4f) by 73% and 38%, respectively, in the absence of significant improvement in the post-ischemic CBF (Fig. 4g) or reduction in fibrin (Fig. 4h) and/or neutrophil deposition (Fig. 4i). In contrast, high dose APC reduced motor neurological score (Fig. 4e) and the volume of brain injury (Fig. 4f) by 91% and 65%, respectively, and also caused a 30% improvement in post-ischemic CBF (Fig. 4e) with significant reductions in fibrin (Fig. 4h) and a moderate reduction in neutrophils (Fig. 4i). Therefore, it appears that APC's neuroprotective activity *in vivo* is distinguishable from APC's anticoagulant activity, as predicted for cytoprotective mechanisms involving the direct effects of APC on PAR-1 and EPCR. These data for different APC doses imply that anticoagulant effects of APC enhance post-ischemic CBF by reducing fibrin accretion but raise a question of whether the observed reduction in the number of leukocytes infiltrating the ischemic brain may be secondary to APC's anti-apoptotic and cytoprotective effects, rather than due to a primary effect of APC on blood-brain barrier trafficking of neutrophils⁵.

APC activity was next investigated in models of neuronal injury. To test whether APC is directly neuronal protective, we studied its effects on N-methyl-D-aspartate (NMDA)-induced apoptosis in cultured mouse cortical neurons²⁹ and on NMDA-induced excitotoxic brain lesions *in vivo* produced by stereotactic NMDA microinjections into mouse caudate nucleus³⁰. Overstimulation of NMDA receptors is implicated in neurodegeneration in stroke and traumatic brain injury and is associated with a number of neurodegenerative disorders including Alzheimer's disease and Huntington's disease^{31,32}. Several mechanisms potentially involved in NMDA-induced neuronal apoptosis include increases in p53 and *Bax*³³⁻³⁵, a proapototic member of the *Bcl*-2 gene family and a transcriptional product of p53¹⁹, activation of caspase-3 signaling^{36,37} resulting in proteolysis of several nuclear substrates, and generation of nitric oxide^{29,30,38}. Since EPCR-dependent signaling by APC through PAR-1 prevents p53-dependent apoptosis of endothelium as shown in Figs. 1-4³⁹, we hypothesized that APC may exert its direct neuronal protective effects on NMDA-induced apoptosis by blocking p53 and caspase-3 signaling through PARs on neurons.

Here, we report that APC blocks NMDA-induced apoptosis in cultured mouse cortical neurons by reducing p53 and caspase-3 pro-apoptotic signaling. Moreover, direct intracerebral infusions of APC significantly reduced NMDA excitotoxic brain lesions in mice. APC's direct neuroprotective effects on perturbed mouse neurons *in vitro* and *in vivo* required PAR-1 and PAR-3 on neurons, suggesting APC may limit neuronal damage in neurodegenerative disorders caused by overstimulation of NMDA receptors.

### EXAMPLE 2:

*Reagents and antibodies.* N-methyl-D-aspartate (NMDA) was purchased from Sigma (St. Louis, MO). Human APC, recombinant mouse APC, protein C zymogen, APC mutants, and mouse IgG against human APC (C3 antibody) were prepared as described^{17,26}. For Western blot analysis or immunostaining we used polyclonal rabbit antibody against human active caspase-3 (1:250, 1 mg/ml; Promega, Madison, WI), human Bcl-2 (1:100, 0.2 mg/ml; Santa Cruz Biotechnology, Santa Cruz, CA), human 53 (1:1000, Cell Signaling, Beverly, MA) and human NMDAξ1 (NR1, 1:1000, 0.2 mg/ml; Santa Cruz Biotechnology, Santa Cruz, CA) that all cross react with the corresponding mouse antigens; mouse NR2A (1:500, 1 mg/ml; Upstate Biotechnology, Lake Placid, NY), mouse Bax (1:100, Chemicon, Temecula, CA) and polyclonal goat antibody against human β-actin (1:2,500, 0.2 mg/ml; Santa Cruz Biotechnology, Santa Cruz, CA) that cross-react with mouse β-actin. All antibodies against PARs were from Santa Cruz Biotechnology (Santa Cruz, CA). Polyclonal rabbit antibodies against human PAR-1 (H-111) and human PAR-3 (H-1 03), monoclonal mouse antibody against human PAR-2 (SAM11) all cross react with the corresponding mouse PARs; this has been confirmed in the present study by positive immunostaining in primary mouse cortical neuronal cultures. Polyclonal goat antibodies against N-terminus of mouse PAR-1 (S-19) and mouse PAR-2 (S-19), and C-terminus of mouse PAR-3 (M-20) and mouse PAR-4 (M-20) were also used as negative controls. PAR-1 and PAR-2 agonist peptides TFLLRNPNDK and SLIGRL were obtained from Ana Spec (San Jose, CA).

*Neuronal culture.* Primary neuronal cultures were established as described⁴⁰. In brief, cerebral cortex was dissected from fetal C57BL/6J mice at 16 days of gestation, treated with trypsin for 10 min at 37°C, and dissociated by trituration. Dissociated cell suspensions were plated at 5 x 10⁵ cells per well on 12-well tissue culture plates or at 4 x 10⁶ cells per dish on 60 mm tissue culture dishes coated with poly-D-lysine, in serum-free Neurobasal medium plus B27 supplement (Gibco BRL, Rockville, MD). The medium suppresses glial growth to less than 2% of the total cell population. The absence of astrocytes was confirmed by the lack of glial fibrillary acidic protein staining. Cultures were maintained in a humidified 5% CO₂ incubator at 37°C for seven days before treatment. Medium was replaced every three days.

*NMDA-induced apoptosis in neuronal culture.* For induction of neuronal apoptosis, cultures were exposed for 10 min to 300 µM NMDA/5 µM glycine in Mg²⁺-free Earle's balanced salt solution (EBSS) as described²⁹. Control cultures were exposed to EBSS alone. After the exposure, cultures were rinsed with EBSS, returned to the original culture medium and incubated with different concentrations of either human APC (1-100 nM) or recombinant mouse APC (1-100 nM) for 0, 3, 6, 12, 24, and 36 hr, protein C zymogen (100 nM), anti-APC IgG (C3, 11 µg/ml), Ser360Ala-APC (100 nM) or boiled APC (100 nM) for 24 hr. Different anti-PARs antibodies (20 µg /ml) were added to the incubation medium simultaneously with mouse recombinant APC (10 nM) after NMDA exposure. TFLLRNPNDK (10 µM) and SLIGRL (100 µM) were added to the incubation medium after NMDA exposure.

*Detection of apoptosis.* Apoptotic cells were visualized by in situ terminal deoxynucleotidyl transferase-mediated digoxigenin-dUTP nick-end labeling (TUNEL) assay according to the manufacturer's instructions (Intergen Company, Purchase, NY). Cells were counterstained with the DNA-binding fluorescent dye, Hoechst 33342 (Molecular Probes, Eugene, OR) at 1 mg/ml for 10 min at room temperature to reveal nuclear morphology. The number of apoptotic cells was expressed as the percentage of TUNEL-positive cells of the total number of nuclei determined by Hoechst staining. The cells were counted in 10 to 20 random fields (30X magnification) by two independent observers blinded to the experimental conditions. The number of cells under basal conditions (vehicle only) was subtracted from the number of apoptotic cells in control and experimental groups.

*Double-labeling for in situ DNA fragmentation and caspase-3.* Subsequent to visualization of fragmented DNA with TUNEL, cells were permeabilized with 0.4% Tween 20 for 30 min and blocked with 10% normal goat serum in PBS for 30 min at room temperature. A primary anti-caspase-3 antibody was applied overnight at 4°C. After washing in PBS three times, cells were incubated with rhodamine conjugated goat anti-rabbit IgG (1:150) for 1 hr at 37°C.

*Analysis of caspase-3 activity.* Proteolytic activity of caspase-3 was analyzed by using an ApoAlert caspase colorimetric assay kit (Clontech, Palo Alto, CA). Cells were washed with PBS and resuspended in cell lysis buffer. Protein (50 µg) was incubated with 50 µM caspase 3 substrate (DEVD-pNA) at 37°C. The colorimetric release of p-nitroaniline from Ac-DEVD-pNA substrate was recorded every 10 min at 405 nm with a microplate reader. Enzymatic activity was expressed in arbitrary units of per mg protein per min.

*Western blot analysis.* Whole cellular and nuclear protein extracts or cell membrane fractions were prepared as described. Protein concentration was determined using Bradford protein assays (Bio-Rad, Hercules, CA); 10-50 µg of protein was analyzed by 10% SDS-PAGE and transferred to nitrocellulose membranes. The membranes were blocked with 5% nonfat milk in TBST (100 mM TRIS, pH 8.0, 1.5 M of NaCl, 0.1 % Tween 20) for 1 hr. The membranes were incubated overnight with primary antibodies in PBST and then washed and incubated with a horseradish peroxidase-conjugated secondary antibody for 1 hr. Immunoreactivity was detected by using the ECL detection system (Amersham, Piscataway, NJ). The relative abundance of each protein was determined by scanning densitometry, using β-actin as an internal control. Data from multiple Western blots (n = 3-5) were averaged for statistical analysis.

*Electrophoretic mobility shift assay (EMSA).* Nuclear proteins were extracted from cortical neuronal cultures at 0, 0.5, 1, 2, 3 and 6 hr after exposure to NMDA using NE-PER^{™} nuclear and cytoplasmic extraction reagents according to manufacturer's instructions (Pierce, Rockford, IL). Human umbilical vein endothelial cells (HUVEC) were exposed to E. coli lipopolysaccharide (LPS) (200 ng/ml) for 4 hr as a positive control. The activation of NF-κB was determined by its binding to the consensus sequence (5'-AGT TGA GGG GAC TTT CCC AGG-3'). Briefly, NF-κB consensus oligonucleotides (Promega, WI) were labeled using digoxigenin gel shift kit (Roche, Indianapolis, IN). Labeled oligonucleotides were incubated with 30 µg nuclear protein extracts at room temperature for 20 min in the reaction buffer (Roche, Indianapolis, IN). Nuclear extracts incubated with NF-κB consensus sequence were run immediately on 4% native polyacrylamide gel in 0.25x TBE. The gel was transferred to Nitron⁺ membrane (Amersham, Piscataway, NJ) and the signal detected according to the manufacturer's manual (Roche, Indianapolis, IN).

*Intrastriatal NMDA microinjections in mice.* All procedures were done as described³⁰ and in accordance with the Animal Care Guidelines at the University of Rochester approved by the National Institutes of Health. C57BL/6J mice, 23-25 g, male were anesthetized with i.p. ketamine (100 mg/kg) and xylazine (10 mg/kg). Animals received microinfusions into the right striatum (0.5 mm anterior, 2.5 mm lateral, 3.2 mm ventral to the bregma) of either vehicle, NMDA (20 nmol in 0.3 µl of PBS, pH 7.4), NMDA + recombinant mouse APC²⁸ (0.002 µg or 0.02 µg or 0.2 µg), NMDA + APC (0.2 µg) + anti-PAR-1 (H-111, 0.2 µg) or anti-PAR-2 (SAM 11, 0.2 µg) or anti-PAR-3 (H-1 03, 0.2 µg). The solutions were infused over 2 min using a microinjection system (World Precision Instruments, Sarasota, FL). The needle was left in place for additional 8 min after the injection as reported³⁰. After 48 hr, mice were sacrificed under deep anesthesia for analysis of excitotoxic lesions. Mice were transcardially perfused with PBS followed by 4% paraformaldehyde in 0.1 M of PBS, pH 7.4. The brains were removed and coronal sections at a 30 µm thickness were prepared using a Vibratome. Every fifth section 1 mm anterior and posterior to the site of injection was stained with cresyl violet. The lesion area was identified by the loss of staining as reported³⁰. The lesion areas were determined by an image analyzer (Image-ProPlus, Media Cybernetics, Silver Spring, MD) and integrated to obtain the volume of injury.

*Statistical analysis.* Data were presented as mean ± SEM. ANOVA was used to determine statistically significant differences. P < 0.05 was considered statistically significant.

Fig. 5a illustrates significant anti-apoptotic effect of human APC (100 nM) on NMDA-perturbed mouse cortical neurons. In response to NMDA, the number of TUNEL-positive cells with apoptotic nuclear changes and the number of caspase-3-positive cells (Figs. 5a-5b) was reduced by greater than 60% by APC. APC reduced in a time-dependent manner the NMDA-induced increase in caspase-3 activity (Fig. 5c) and the number of TUNEL-positive cells (Fig. 5d). Fig. 5e shows dose-dependent neuronal protection by human and mouse recombinant APC. The IC₅₀ values for reducing neuronal apoptosis for human and mouse APC in the NMDA model were 49 and 5 nM, respectively, confirming significantly higher efficacy of the species homologous mouse APC consistent with the above and a recent report in a mouse stroke model³⁹.

To elucidate how APC interferes with the molecular cascades involved in NMDA-induced neuronal apoptosis, first, we demonstrated that APC significantly reduces up to 60% the increased levels of the tumor suppressor protein p53 in nuclear extracts of NMDA-treated neurons between 3 hr and 24 hr (Figs. 6a and 6d), and reduces the levels of p53 mRNA transcripts (Fig. 6b), consistent with its effects in ischemic brain endothelium³⁹. Phosphorylation of p53 on Ser20 or Ser15, which would stabilize the protein by preventing its proteosomal degradation by precluding its binding to the oncoprotein, murine double minute-2 (Mdm2), was undetectable either in nuclear extracts or whole cell homogenates. Changes in Mdm2 protein in response to NMDA were undetectable, and direct effects of APC on Mdm2 protein were not observed. Figs. 6c-6d confirm that an increased pro-apoptotic *Bax*/*Bcl-2* ratio is implicated in NMDA-induced neuronal injury³⁵. Consistent with p53 blockage, APC blunted the increases in *Bax* and the decreases of *Bcl-2* (Figs. 6c-6d), thus favorably altering the *Bax*/*Bcl-2* ratio.

Transcriptionally-dependent p53 induction is involved in neuronal apoptosis triggered by excitatory amino acids³⁴ and NMDA (Fig. 6b), and nitric oxide evokes p53 accumulation and apoptosis⁴¹. Although present findings provide definitive evidence that APC interferes with NMDA-induced apoptosis by blocking p53 and caspase-3 pro-apoptotic signaling and by normalizing the pro-apoptotic *Bax*/*Bcl-2* ratio in stressed neurons, the exact relationship between APC's anti-apoptotic mechanisms and NMDA-induced nitric oxide neuronal toxicity remains to be defined.

Since NMDA may increase the levels of nuclear factor κB (NF-κB)⁴² that can be either anti-apoptotic or pro-apoptotic⁴³, we tested whether the observed changes in p53 expression are downstream to NF-κB. NMDA did not induce NF-κB translocation into the nucleus in cortical cells (Fig. 6e) whereas in the positive control, *E*. *coli* lipopolysacharide did cause NF-κB translocation in umbilical vein endothelium (Fig. 6e). This confirms that in the present NMDA-induced apoptosis model, NF-κB nuclear translocation is not involved in neuronal apoptosis.

We tested whether APC can affect NMDA receptor structure by proteolysis as reported for tissue plasminogen activator (tPA)⁴⁴. In contrast to tPA, APC did not cleave either the NR1 or NR2A subunits of NMDA receptors (Fig. 6f), confirming that APC does not modify the properties of NMDA receptors and suggesting APC acts downstream from NMDA receptor stimulation. NMDA receptors mediate ischemic brain injury, but blocking these receptors can be deleterious to animals and humans^{45,46}. Thus, interfering with NMDA-induced apoptosis downstream to the NMDA receptors, by using APC to block NMDA-induced p53 and caspase-3 pro-apoptotic signaling and/or by limiting generation of nitric oxide by uncoupling the NR2 subunits of NMDA receptors from neuronal nitric oxide synthase⁴⁷, offers an attractive brain protection strategy.

To define the specificity of APC's neuroprotection, we demonstrated that neither mutant Ser360Ala-APC nor protein C zymogen could protect neurons from NMDA-induced apoptosis, and that heat denaturation or anti-APC IgG abrogated APC's activity (Fig. 7a). This confirms that the active site serine of APC is necessary for neuronal protection, raising the possibility that PARs may be involved. To test the role of PARs, we studied the neuronal protective effects of mouse recombinant APC in the presence of various anti-PAR antibodies (Fig. 7b). All four PARs are present in rodent CNS⁴⁸, as we confirmed by positive immunostaining of PAR-1, PAR-2, PAR-3 and PAR-4 on mouse cortical neurons. Antibody blockage of the PAR-1 cleavage site, but not of the PAR-2 cleavage site, caused significant (about 70%) loss of APC-mediated neuronal protection (Fig. 7b). In negative controls, antibodies against the N-terminal, cleavage-independent regions of PAR-1 and PAR-2, or against the C-terminus of PAR-4, were without effect (Fig. 7b). An antibody against the extracellular N-terminal 103 amino acids of PAR-3 significantly blocked APC-mediated neuronal protection (about 65%), in contrast to a control anti-PAR-3 antibody against the C-terminus of PAR-3 (Fig. 7b). The combination of antibodies that block activation of PAR-1 and PAR-3 completely abrogated APC's neuronal protective effects (Fig. 7b). In studies of thrombin signaling in mouse platelets, PAR-3 does not provide thrombin signaling but rather serves as a cofactor for PAR-4 activation by thrombin⁴⁹. Thus, the present results suggest that APC binds to PAR-3 and activates PAR-1 to rescue neurons from NMDA-induced toxicity.

To test further the role of PAR-1, we demonstrated that PAR-1 agonist peptide (TFLLRNPNDK) but not the PAR-2 agonist peptide (SLIGRL) protected neurons against NMDA-induced apoptosis (Fig. 7b). PAR-1 agonist peptide and thrombin at relatively higher concentrations can kill neurons^{50,51}. But at concentrations comparable to those used in the present study, the PAR-1 agonist can protect cortical rat neurons and astrocytes from hypoglycemia and oxygen/glucose deprivation^{50,51}, as seen here for NMDA toxicity. Thus, activation of PAR-1 in neurons may be anti-apoptotic, as in the case of APC and low dose thrombin. The pro-apoptotic activity of higher levels of thrombin may involve the action of thrombin on substrates other than PAR-1 (e.g., PAR-4) and/or differences in amplitude and duration of PAR-1 signaling. APC cleaves a synthetic PAR-1 N-terminal polypeptide at Arg 41, the thrombin cleavage site, at a rate 5,000 times slower than thrombin⁵². Presumably, binding of APC to plasma membrane phospholipids or EPCR near the extracellular N-terminal tail of PAR-1 accelerates APC's cleavage at Arg 41.

To determine whether APC's *in vivo* neuroprotection has similar requirements for PAR-1 and PAR-3 as in cultured neurons, we studied the effects of APC on NMDA-induced excitotoxic lesions in mouse brain using stereotactic striatal injections of NMDA³⁰. Administration of mouse APC (0.2 µg) to mice with NDMA-induced brain injury significantly reduced the lesion volume at 48 hr by greater than 70% (Figs. 7c-7d); the effect of APC was dose-dependent (Fig. 7d) similar to the *in vitro* results (Fig. 7e). There was greater than 70% loss of APC neuroprotective effect *in vivo* in mice treated with APC plus anti-PAR-1 antibody (Fig. 7e) and greater than 65% loss of APC-mediated neuroprotection in the presence of anti-PAR-3 antibody. These studies confirmed that PAR-1 and/or PAR-3 significantly contribute to APC's neuronal protection both *in vitro* and *in vivo.*

Finally, we confirmed that APC mutants which lack wild-type levels of anticoagulant activity may retain normal neuroprotective activity. Human APC protease domain mutants with low anticoagulant activity (see locations shown in Fig. 8) were assayed for their anti-apoptotic activity using NMDA-perturbed mouse neurons *in vitro* and *in vivo* as well as hypoxic human brain endothelial cells *in vitro* (Figs. 9a-9f). The procedures and animal models used to assay neuroprotective activity of APC mutants were as described above. Two human APC mutants with alanine substitutions in either loop 37 (KKK191-193AAA, "3K3A-APC") or the Ca⁺⁺-binding loop (RR229/230AA, "229/30-APC") had neuroprotective activity like recombinant wild-type human APC ("rwt-APC") in all three models studied, whereas the APC mutants had less than 10% and about 5% of the anticoagulant activity of wild-type human APC. None of these mutations affected APC amidolytic activity. Thus, these human APC mutants with reduced anticoagulant activity retain wild-type levels of *in vitro* and *in vivo* neuroprotective activity which act directly on brain cells. Such APC mutants are termed "functional mutants" because they are selectively deficient in APC's anticoagulant activity and therefore may have less risk of bleeding. The IC₅₀ values of 3K3A-APC and 229/30-APC on NMDA-treated mouse neurons and hypoxic human BEC were approximately 11 nM and 18 nM, respectively, and around 10-12 nM for both.

In summary, the present findings indicate that APC has direct neuronal protective properties that do not depend on its systemic actions, and that APC prevents neuronal apoptosis by directly acting on perturbed neurons. In contrast, clot-dissolving tPA protease exerts direct brain cell neurotoxicity^{24,44}. Thus, APC acting via PAR-1 and PAR-3 may critically limit neuronal damage by preventing NMDA-induced neuronal apoptosis in neurodegenerative disorders associated with overstimulation of NMDA receptors.

### REFERENCES

1. Griffin et al. Activated protein C: Potential therapy for severe sepsis, thrombosis, and stroke. Semin. Hematol. 39:197-205 (2002).
2. Griffin. Blood coagulation. The thrombin paradox. Nature 378:337-338 (1995).
3. Esmon et al. Endothelial protein C receptor. Thromb. Haemost. 82:251-258 (1999).
4. Mizutani et al. Activated protein C reduces ischemia/reperfusion-induced renal injury in rats by inhibiting leukocyte activation. Blood 95:3781-3787 (2000).
5. Shibata et al. Anti-inflammatory, antithrombotic, and neuroprotective effects of activated protein C in a murine model of focal ischemic stroke. Circulation 103:1799-1805 (2001).
6. Bernard et al. Efficacy and safety of recombinant human activated protein C for severe sepsis. New Engl. J. Med. 344:699-709 (2001).
7. Folsom et al. Prospective study of markers of hemostatic function with risk of ischemic stroke. The Atherosclerosis Risk in Communities (ARIC) Study Investigators. Circulation 100:736-742 (1999).
8. Joyce et al. Gene expression profile of antithrombotic protein C defines new mechanisms modulating inflammation and apoptosis. J. Biol. Chem. 276:11199-11203 (2001).
9. Riewald et al. Activation of endothelial cell protease activated receptor 1 by the protein C pathway. Science 296:1880-1882 (2002).
10.Castellino et al. Mice with a severe deficiency of the endothelial protein C receptor gene develop, survive, and reproduce normally, and do not present with enhanced arterial thrombosis after challenge. Thromb. Haemost. 88:462-472 (2002).
11. Oren. Regulation of the p53 tumor suppressor protein. J. Biol. Chem. 274:36031-36034 (1999).
12. Carr. Cell cycle. Piecing together the p53 puzzle. Science 287:1765-1766 (2000).
13.Ogawara et al. Akt enhances Mdm2-mediated ubiquitination and degradation of p53. J. Biol. Chem. 277:21843-21850 (2002).
14.Stempien-Otero et al. Mechanisms of hypoxia-induced endothelial cell death. Role of p53 in apoptosis. J. Biol. Chem. 274:8039-8045 (1999).
15. Kimura et al. Orphan G protein-coupled receptor, GPR41, induces apoptosis via a p53/Bax pathway during ischemic hypoxia and reoxygenation. J. Biol. Chem. 276:26453-26460 (2001).
16.Zhu et al. p38 Mitogen-activated protein kinase mediates hypoxic regulation of Mdm2 and p53 in neurons. J. Biol. Chem. 277:22909-22914 (2002).
17. Gale et al. Nonenzymatic anticoagulant activity of the mutant serine protease Ser360Ala-activated protein C mediated by factor Va. Protein Sci. 6:132-140 (1997).
18.Ye et al. The endothelial cell protein C receptor (EPCR) functions as a primary receptor for protein C activation on endothelial cells in arteries, veins, and capillaries. Biochem. Biophys. Res. Commun. 259:671-677 (1999).
19. Miyashita et al. Tumor suppressor p53 is a direct transcriptional activator of the human bax gene. Cell 80:293-299 (1995).
20. Yamamoto et al. Contribution of Bcl-2, but not Bcl-xL and Bax, to antiapoptotic actions of hepatocyte growth factor in hypoxia-conditioned human endothelial cells. Hypertension 37:1341-1348 (2001).
21. Faleiro et al. Caspases disrupt the nuclear-cytoplasmic barrier. J. Cell Biol. 151:951-959 (2000).
22.Ackerman et al. The role of antiapoptotic Bel-2 family members in endothelial apoptosis elucidated with antisense oligonucleotides. J. Biol. Chem. 274:11245-11252 (1999).
23. Hoffman et al. Hypoxia-induced upregulation of eNOS gene expression is redox-sensitive: a comparison between hypoxia and inhibitors of cell metabolism. J. Cell. Physiol. 188:33-44 (2001).
24.Wang et al. Tissue plasminogen activator (tPA) increases neuronal damage after focal cerebral ischemia in wild-type and tPA-deficient mice. Nat. Med. 4:228-231 (1998).
25. Flavin et al. Microglial tissue plasminogen activator (tPA) triggers neuronal apoptosis in vitro. Glia 29:347-354 (2000).
26. Gruber & Griffin. Direct detection of activated protein C in blood from human subjects. Blood 79:2340-2348 (1992).
27. Mackic et al. Human blood-brain barrier receptors for Alzheimer's amyloid-beta 1- 40. Asymmetrical binding, endocytosis, and transcytosis at the apical side of brain microvascular endothelial cell monolayer. J. Clin. Invest. 102:734-743 (1998).
28. Fernandez et al. Recombinant murine-activated protein C in a murine ischemic stroke model. Blood Cell. Mol. Dis., 30:271-276 (2003)
29. Bonfoco et al. Apoptosis and necrosis: Two distinct events induced, respectively, by mild and intense insults with N-methyl-D-aspartate or nitric oxide/superoxide in cortical cell cultures. Proc. Natl. Acad. Sci. U.S.A. 92:7162-7166 (1995).
30.Ayata et al. Mechanisms of reduced striatal NMDA excitotoxicity in type I nitric oxide synthase knock-out mice. J. Neurosci. 17:6908-6917 (1997).
31. Kemp et al. NMDA receptor pathways as drug targets. Nature Neurosci. 5:1039-1042 (2002).
32. Le et al. Potential and current use of N-methyl-D-aspartate (NMDA) receptor antagonists in diseases of aging. Drugs & Aging 18:717-724 (2001).
33. Sakhi et al. Induction of tumor suppressor p53 and DNA fragmentation in organotypic hippocampal cultures following excitotoxin treatment. Exp. Neurol. 145:81-88 (1997).
34. Uberti et al. Induction of tumour-suppressor phosphoprotein p53 in the apoptosis of cultured rat cerebellar neurons triggered by excitatory amino acids. Eur. J. Neurosci.10:246-254 (1998).
35. Djebaili et al. p53 and Bax implication in NMDA induced-apoptosis in mouse hippocampus. Neuroreport. 11:2973-2976 (2000).
36. Du et al. Activation of a caspase 3-related cysteine protease is required for glutamate-mediated apoptosis of cultured cerebellar granule neurons. Proc. Natl. Acad. Sci. U.S.A. 94:1657-11662 (1997).
37. Okamoto et al. Dominant-interfering forms of MEF2 generated by caspase cleavage contribute to NMDA-induced neuronal apoptosis. Proc. Natl. Acad. Sci. U.S.A. 99:3974-3979 (2002).
38. Dawson et al. Nitric oxide mediates glutamate neurotoxicity in primary cortical cultures. Proc. Natl. Acad. Sci. U.S.A. 88:6368-6371 (1991).
39.Cheng et al. Activated protein C blocks p53-mediated apoptosis in ischemic human brain endothelium and is neuroprotective. Nature Med. 9:338-342 (2003).
40. Brewer et al. Optimized survival of hippocampal neurons in B27-supplemented Neurobasal, a new serum-free medium combination. J. Neurosci. Res. 35:567-576 (1993).
41. Brüne et al. Nitric oxide evoked p53-accumulation and apoptosis. Toxicology Letters 139:119-123 (2003).
42. McInnis et al. The role of superoxide and nuclear factor-κB signaling in N-methyl-D-aspartate-induced necrosis and apoptosis. J. Pharmacol. Exp. Ther. 301:478-487 (2002).
43. Ryan et al. Role of NF-κB in p53-mediated programmed cell death. Nature 404:892-897 (2000).
44. Nicole et al. The proteolytic activity of tissue-plasminogen activator enhances NMDA receptor-mediated signaling. Nature Med. 7:59-64 (2001).
45. Davis et al. Selfotel in acute ischemic stroke: Possible neurotoxic effects of an NMDA antagonist. Stroke 31:347-354 (2000).
46. Morris et al. Failure of the competitive N-methyl-D-aspartate antagonist Selfotel (CGS 19755) in the treatment of severe head injury: Results of two Phase III clinical trials. J. Neurosurg. 91:737-743 (1999).
47.Aarts et al. Treatment of ischemic brain damage by perturbing NMDA receptor-PSD-95 protein interactions. Science 298:846-850 (2002).
48. Striggow et al. Four different types of protease-activated receptors are widely expressed in the brain and are up-regulated in hippocampus by severe ischemia. Eur. J. Neurosci. 14:595-608 (2001).
49. Nakanishi-Matsui et al. PAR3 is a cofactor for PAR4 activation by thrombin. Nature 404:609-613 (2000).
50. Striggow et al. The protease thrombin is an endogenous mediator of hippocampal neuroprotection against ischemia at low concentrations but causes degeneration at high concentrations. Proc. Natl. Acad. Sci. U.S.A. 97:2264-2269 (2000).
51. Donovan et al. Signaling pathways involved in thrombin-induced cell protection. J. Biol. Chem. 273:12746-12752 (1998).
52. Kuliopulos et al. Plasmin desensitization of the PAR1 thrombin receptor: Kinetics, sites of truncation, and implications for thrombolytic therapy. Biochemistry 38:4572-4585 (1999).

## Claims

1. A pharmaceutical composition comprising at least one functional variant of an activated human protein C (APC) or a human protein C for use in the treatment of a neurodegenerative disease selected from the group consisting of ischemia and stroke, wherein the functional variant is comprised of at least the mutation KKK191-193AAA, **characterized in that** the functional variant is present in an effective amount to provide neuroprotection for stressed or injured cells in a subject.

2. The pharmaceutical composition of claim 1 which results in at least reduced or insignificant systemic anticoagulation when administered to the subject.

3. The pharmaceutical composition of claim 1 or 2, wherein the unit dose is administered to the subject in less than 72 hours.

4. Use of at least one functional variant of an activated human protein C (APC) or a human protein C, wherein the functional variant is comprised of at least the mutation KKK191-193AAA, for the manufacture of a pharmaceutical composition to treat a neurodegenerative disease selected from the group consisting of ischemia and stroke, which is comprised of administering an effective amount of the pharmaceutical composition to a subject such that at least one effect of stress or injury is improved in one or more cell types of the subject; wherein the one or more cell types are in the subject's brain; and wherein the subject is a human.

## Patentansprüche

1. Pharmazeutische Zusammensetzung, die mindestens eine funktionale Variante eines aktivierten menschlichen Proteins C (APC) oder ein menschliches Protein C zur Verwendung in der Behandlung einer neurodegenerativen Erkrankung aus der folgenden Gruppe umfasst: Ischämie und Schlaganfall, wobei die funktionale Variante mindestens aus der Mutation KKK191-193AAA besteht, **dadurch gekennzeichnet, dass** die funktionale Variante in einer effektiven Menge vorliegt, um eine Neuroprotektion für gestresste oder verletzte Zellen in einem Subjekt zur Verfügung zu stellen.

2. Pharmazeutische Zusammensetzung gemäß Anspruch 1, die zu mindestens einer verringerten oder insignifikanten systemischen Antikoagulation führt, wenn sie dem Subjekt verabreicht wird.

3. Pharmazeutische Zusammensetzung gemäß Anspruch 1 oder 2, wobei die Einzeldosis dem Subjekt in weniger als 72 Stunden verabreicht wird.

4. Verwendung von mindestens einer funktionalen Variante eines aktivierten menschlichen Proteins C (APC) oder eines menschlichen Proteins C, wobei die funktionale Variante mindestens aus der Mutation KKK191-193AAA besteht, zur Herstellung einer pharmazeutischen Zusammensetzung zur Behandlung einer neurodegenerativen Erkrankung aus der folgenden Gruppe: Ischämie und Schlaganfall, die umfasst: Verabreichen einer effektiven Menge der pharmazeutischen Zusammensetzung an ein Subjekt, so dass mindestens eine Wirkung von Stress oder Verletzung in einem oder mehreren Zelltypen des Subjekts verbessert wird; wobei sich der eine oder die mehreren Zelltypen in dem Gehirn des Subjekts befinden; und wobei das Subjekt ein Mensch ist.

## Revendications

1. Composition pharmaceutique comprenant au moins un variant fonctionnel d'une protéine C humaine activée (APC) ou d'une protéine C humaine, pour utilisation dans le traitement d'une affection neurodégénérative choisie parmi une ischémie et une attaque, dans laquelle le variant fonctionnel porte au moins la mutation KKK191-193AAA, **caractérisée en ce que** le variant fonctionnel s'y trouve présent en une quantité efficace pour apporter une neuro-protection à des cellules soumises à un stress ou lésées chez un sujet.

2. Composition pharmaceutique conforme à la revendication 1, qui, lorsqu'on l'administre à un sujet, fait preuve d'une activité anticoagulation systémique au moins réduite ou insignifiante.

3. Composition pharmaceutique conforme à la revendication 1 ou 2, dont on administre une dose unitaire au sujet moins de 72 heures après.

4. Utilisation d'au moins un variant fonctionnel d'une protéine C humaine activée (APC) ou d'une protéine C humaine, lequel variant fonctionnel porte au moins la mutation KKK191-193AAA, pour la fabrication d'une composition pharmaceutique conçue pour le traitement d'une affection neurodégénérative choisie parmi une ischémie et une attaque, consistant à administrer à un sujet la composition pharmaceutique en une quantité efficace de manière à ce que soit atténué, chez le sujet, au moins un effet d'un stress ou d'une lésion au sein de cellules d'un type ou de plusieurs types, étant entendu que les cellules d'un type ou de plusieurs types se trouvent dans le cerveau dudit sujet, et que ledit sujet est un humain.
